# EUROPEAN PATENT APPLICATION

(11) **EP 4 567 463 A1**
(43) Date of publication of application: **11.06.2025**
(21) Application number: 24164035.8
(22) Date of filing: 18.03.2024
(51) Int. Cl.: G01S 7/52, G01S 15/89

(54) **PROBE AND METHOD OF CONTROLLING THE PROBE**

(30) Priority: 05.12.2023 KR 20230174933
(71) Applicant: Samsung Medison Co., Ltd., Hongcheon-gun, Gangwon-do 25108 (KR)
(72) Inventor: Kim, Mose, Seoul (KR); Lee, Yoonchang, Seoul (KR)
(74) Representative: Frey, Sven Holger

(57) **Abstract**

Provided are a probe and a control method of the probe. In detail, provided are a probe and a control method of the probe, the probe including a plurality of transducers having parallel center axes, a beamformer configured to control the plurality of transducers, a communication module, and a processor, wherein the processor is configured to transmit a transmission signal by using at least one of the plurality of transducers, receive, by using the plurality of transducers, a plurality of frequency components generated due to the transmission signal, generate ultrasound data, based on the plurality of frequency components received by the plurality of transducers, and control the communication module to transmit the ultrasound data to an ultrasound imaging device.

## Description

### CROSS-REFERENCE TO RELATED APPLICATION

This application is based on and claims priority under 35 U.S.C. §119 to Korean Patent Application No. 1 0-2023-0174933, filed on December 5, 2023, in the Korean Intellectual Property Office, the disclosure of which is incorporated by reference herein in its entirety.

### BACKGROUND

### 1. Field

The disclosure relates to a probe and a method of controlling the probe. In particular, the disclosure relates to a method of obtaining ultrasound data by using a probe including a plurality of transducers.

### 2. Description of the Related Art

Recently, various medical imaging devices for imaging and obtaining information about tissues and organs of the human body have been widely used for the purposes of early diagnosis and treatment of diseases. Representative examples of the medical imaging devices may include an ultrasound imaging device, a computed tomography (CT) apparatus, and a magnetic resonance imaging (MRI) apparatus.

An ultrasound imaging device irradiates an ultrasound signal generated from a transducer of a probe to an object, receives information from a signal reflected from the object, and noninvasively obtains at least one image of a region (e.g., soft tissue or blood flow) inside the object. The ultrasound imaging device is used for medical purposes, such as observation of an inside part of an object, detection of foreign substances, injury measurement, etc. As the ultrasound imaging device has higher stability than other imaging devices using X-rays, is capable of displaying an image in real time, and is free from radiation exposure, the ultrasound imaging device is widely used along with other imaging devices.

The probe of the ultrasound imaging device may transmit a transmission signal to an object coated with a contrast agent, and may receive a reception signal reflected from the object. The reception signal reflected from the object coated with the contrast agent may include a plurality of frequency components. Due to a physical feature of the contrast agent, a plurality of harmonic components may occur when the reception signal is reflected. The transducer of the probe may receive only a frequency component that belongs to a designated frequency band from among the plurality of frequency components. As some of the plurality of frequency components are not received, a quality of an obtained ultrasound image may deteriorate.

### SUMMARY

Additional aspects will be set forth in part in the description which follows and, in part, will be apparent from the description, or may be learned by practice of the presented embodiments of the disclosure.

According to an embodiment, a probe includes a plurality of transducers having parallel center axes and configured to receive an ultrasound signal of different frequency ranges, a beamformer configured to control the plurality of transducers, a communication module, and a processor. According to an embodiment, the processor may be configured to transmit a transmission signal by using at least one of the plurality of transducers. According to an embodiment, the processor may be configured to transmit a transmission signal by receive, by using the plurality of transducers, a plurality of frequency components that are generated due to the transmission signal and are included in the different frequency ranges. According to an embodiment, the processor may be configured to generate ultrasound data, based on the plurality of frequency components received by the plurality of transducers. According to an embodiment, the processor may be configured to control the communication module to transmit the ultrasound data to an ultrasound imaging device.

According to an embodiment, a control method of a probe includes transmitting, by the probe, a transmission signal by using at least one of a plurality of transducers having parallel center axes and configured to receive an ultrasound signal of different frequency ranges. According to an embodiment, the control method of the probe may include receiving, by using the plurality of transducers, a plurality of frequency components that are generated due to the transmission signal and are included in the different frequency ranges. According to an embodiment, the control method of the probe may include generating ultrasound data, based on the plurality of frequency components received by the plurality of transducers. According to an embodiment, the control method of the probe may include transmitting the ultrasound data to an ultrasound imaging device.

### BRIEF DESCRIPTION OF THE DRAWINGS

The disclosure will become apparent and more readily appreciated from the following description, taken in conjunction with the accompanying drawings wherein like reference numerals denote like structural elements.

The above and other aspects, features, and advantages of certain embodiments of the disclosure will be more apparent from the following description taken in conjunction with the accompanying drawings, in which:
FIGS. 1A and 1B are block diagrams each illustrating a configuration of an ultrasound imaging system according to an embodiment;
FIGS. 2A, 2B, 2C, and 2D are diagrams illustrating ultrasound imaging devices according to an embodiment;
FIG. 3 is a block diagram of a probe according to an embodiment;
FIG. 4 is a diagram illustrating a first transducer and a second transducer of a probe, according to an embodiment;
FIG. 5A is a diagram illustrating a plurality of frequency components received by a probe, a first reception frequency range of the probe, and a second reception frequency range of the probe, according to an embodiment;
FIG. 5B is a diagram illustrating a plurality of frequency components received by a probe, a first reception frequency range of the probe, and a second reception frequency range of the probe, according to an embodiment;
FIG. 6 is a diagram illustrating a plurality of frequency components received by a probe, a first reception frequency range of the probe, and a second reception frequency range of the probe, according to an embodiment;
FIG. 7 is a flowchart illustrating a control method of a probe, according to an embodiment;
FIG. 8 illustrates that a probe receives and synthesizes a low frequency reception signal and a high frequency reception signal due to a transmission signal of a first transducer and displays a result of the synthesis, according to an embodiment;
FIG. 9 illustrates that a probe receives each of a low frequency reception signal and a high frequency reception signal, thereby generating and displaying different images, according to an embodiment;
FIG. 10 illustrates an external device, which received ultrasound data from a probe, displaying a high-penetration image and a high-resolution image, according to an embodiment;
FIG. 11 illustrates an external device, which received ultrasound data from a probe, displaying a synthesis image, according to an embodiment;
FIG. 12 illustrates that, when each of a first transducer and a second transducer of a probe receive and synthesize a plurality of frequency components, a difference between a first time point of the first transducer and a second time point of the second transducer is compensated, according to an embodiment;
FIG. 13 illustrates a structure in which a first transducer and a second transducer of a probe are combined to be parallel to each other, according to an embodiment;
FIG. 14 illustrates a structure in which a first transducer and a second transducer of a probe are combined to be parallel, according to an embodiment;
FIG. 15 is a block diagram illustrating that two beamformer modules included in a beamformer of a probe respectively control two transducers, according to an embodiment;
FIG. 16 is a block diagram illustrating that two beamformer modules included in a beamformer of a probe respectively control two transducers, according to an embodiment;
FIG. 17 is a block diagram illustrating that a first channel module and a second channel module, which are connected to a beamformer of a probe, respectively control two transducers, according to an embodiment;
FIG. 18 is a block diagram illustrating that a first channel module and a second channel module, which are connected to a beamformer of a probe, respectively control two transducers, according to an embodiment;
FIG. 19 illustrates that a probe receives each of a fundamental reception signal and a sub-harmonic reception signal, and thus, generates and displays a high-resolution image and a high-penetration image, according to an embodiment;
FIG. 20 illustrates that a probe receives and synthesizes a low frequency reception signal and a high frequency reception signal due to a first transmission signal of a first transducer and a second transmission signal of a second transducer and displays a result of the synthesis, according to an embodiment; and
FIG. 21 is a flowchart illustrating that a probe generates a high-penetration image and a high-resolution image, according to an embodiment.

### DETAILED DESCRIPTION

Embodiments and principles thereof will now be provided to clearly define the scope of claims of the disclosure and for one of ordinary skill in the art to be able to perform the embodiments. The embodiments may be implemented in many different forms.

Throughout the specification, like reference numerals denote like elements. Not all elements of the embodiments are described in the specification, and general features in the art or redundant features among the embodiments are omitted. Throughout the specification, a term such as "module" or "unit" may be implemented as one of or a combination of at least two of software, hardware, and firmware, and in some embodiments, a plurality of modules or a plurality of units may be implemented as one element, or a module or a unit may include a plurality of elements.

A singular form of a noun corresponding to an item may include the item or a plurality of the items, unless the context clearly indicates otherwise.

In the disclosure, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases.

The term "and/or" includes any and all combinations of one or more of the associated listed elements.

The terms as "1st" and "2nd," or "first" and "second" may be used to simply distinguish a corresponding element from another, and does not limit the elements in other aspect (e.g., importance or order).

Also, the terms "front surface", "rear surface", "top surface", "bottom surface", "side surface", "left side", "right side", "upper", "lower" etc. used in the disclosure are defined based on drawings, and a shape and a location of each element are not limited due to the terms.

The terms such as "including", "comprising" or "having," etc., are intended to indicate the existence of the features, numbers, steps, actions, elements, parts, or combinations thereof disclosed in the disclosure, and are not intended to preclude the possibility that one or more other features, numbers, steps, actions, elements, parts, or combinations thereof may exist or may be added.

It will be understood that when an element is referred to as being "connected to", "coupled to", "supported by" or "in contact with" another element, it can be directly connected to, coupled to, supported by, or in contact with the other element, or it can be indirectly connected to, coupled to, supported by, or in contact with the other element via a third element.

It will be understood that when an element is referred to as being "on" or "over" another element, the element may contact the other element, or intervening elements may be present.

Hereinafter, an ultrasonic device according to various embodiments will now be described in detail with reference to attached drawings. In description with reference to attached drawings, elements that are the same or are in correspondence are rendered the same reference numeral, and redundant explanations are omitted.

In the disclosure, an image may include a medical image obtained by a medical imaging apparatus such as magnetic resonance imaging (MRI) apparatus, a computed tomography (CT) apparatus, an ultrasound imaging device, or an X-ray imaging apparatus.

In the disclosure, an "object" may be a target to be imaged and may include a human, an animal, or a part of a human or animal. For example, an object may include a body part (e.g., an organ) or a phantom.

In the disclosure, an "ultrasound image" refers to an image of an object generated or processed based on an ultrasound signal transmitted to the object and reflected therefrom.

Hereinafter, embodiments will be described in detail with reference to the drawings.

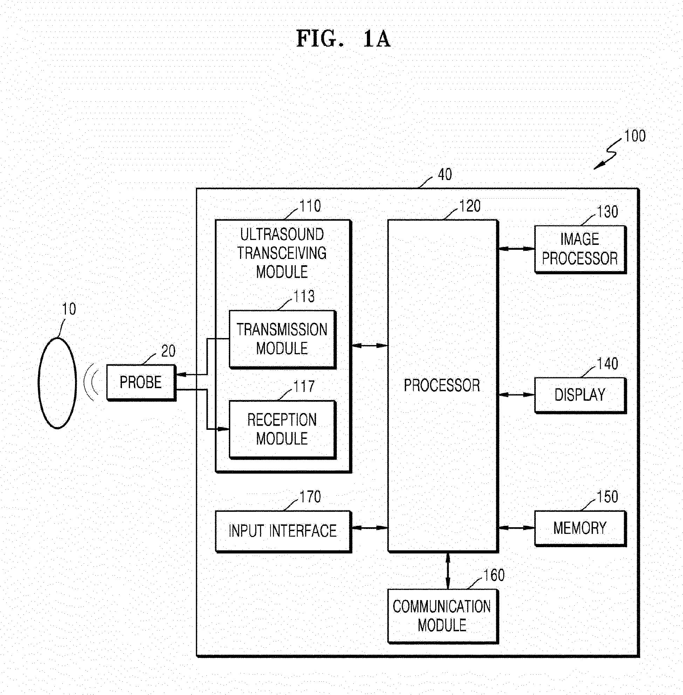

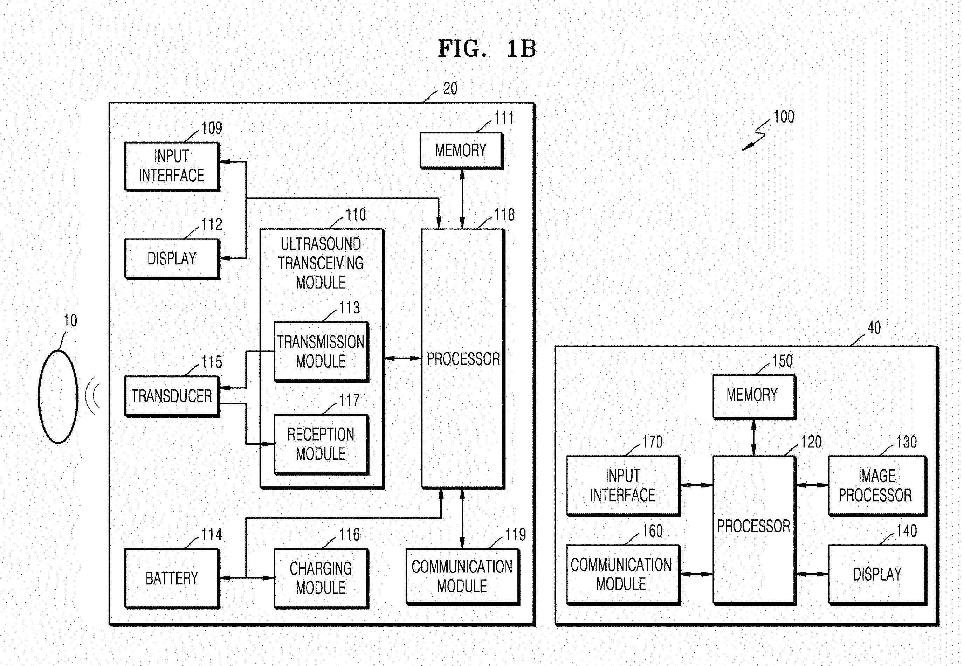

FIGS. 1A and 1B are block diagrams illustrating a configuration of an ultrasound imaging system 100, according to an embodiment.

Referring to FIGS. 1A and 1B, the ultrasound imaging system 100 may include a probe 20 and an ultrasound imaging device 40.

The ultrasound imaging device 40 may be implemented not only as a cart type but also as a portable type. Examples of a portable ultrasound imaging device may include, but are not limited to, a smart phone, a laptop computer, a personal digital assistant (PDA), and a tablet personal computer (PC) that include a probe and an application. The ultrasound imaging device 40 may be integrally implemented with a probe.

The probe 20 may include a wired probe connected to the ultrasound imaging device 40 by wire so as to communicate with the ultrasound imaging device 40 by wire, a wireless probe connected to the ultrasound imaging device 40 by wirelessly so as to communicate with the ultrasound imaging device 40 by wirelessly, and/or a hybrid probe connected to the ultrasound imaging device 40 by wire or wirelessly so as to communicate with the ultrasound imaging device 40 by wire or wirelessly.

According to various embodiments, as illustrated in FIG. 1A, the ultrasound imaging device 40 may include an ultrasound transceiving module 110, and as illustrated in FIG. 1B, the probe 20 may include the ultrasound transceiving module 110. According to various embodiments, it is possible that each of the ultrasound imaging device 40 and the probe 20 includes the ultrasound transceiving module 110.

According to various embodiments, the probe 20 may further include at least one or a combination of an image processor 130, a display 140, or an input interface 170. In the disclosure, descriptions of the ultrasound transceiving module 110, the image processor 130, the display 140, or the input interface 170 may be applied to the ultrasound transceiving module 110, the image processor 130, the display 140, or the input interface 170, which is included in the probe 20.

FIG. 1A is a block diagram illustrating the configuration of the ultrasound imaging system 100 of a case in which the probe 20 is the wired probe or the hybrid probe.

The probe 20 may include a plurality of transducers. The plurality of transducers may be aligned in a preset array to be implemented as a transducer array. The transducer array may correspond to a one-dimensional (1D) array or a two-dimensional (2D) array. The plurality of transducers may transmit an ultrasound signal to an object 10, according to a transmission signal applied from a transmission module 113. The plurality of transducers may generate a reception signal by receiving an ultrasound signal (an echo signal) reflected from the object 10. Also, the probe 20 may be integrally implemented with the ultrasound imaging device 40, or may be implemented as a separate unit connected to the ultrasound imaging device 40 by wire. Also, according to an implementation, the ultrasound imaging device 40 may be connected to one or more probes 20.

When the probe 20 is the wired probe or the hybrid probe, the probe 20 may include a cable and a connector which are connectable to a connector of the ultrasound imaging device 40.

According to an embodiment, the probe 20 may be implemented as a 2D probe. When the probe 20 is implemented as the 2D probe, the plurality of transducers included in the probe 20 may be two-dimensionally aligned to form a 2D transducer array.

For example, the 2D transducer array may include a plurality of subarrays in a first direction and a second direction different from the first direction, each of the subarrays including a plurality of transducers aligned in the first direction.

Also, when the probe 20 according to an embodiment is implemented as the 2D probe, the ultrasound transceiving module 110 may include at least one of an analog beamformer or a digital beamformer. Also, according to an embodiment, the 2D probe may include at least one or a combination of the analog beamformer or the digital beamformer, according to an implementation.

In consideration of positions and focal points of the plurality of transducers 115 included in the probe 20, a processor 120 controls the transmission module 113 to generate transmission signals to be respectively applied to the plurality of transducers 115.

The processor 120 may control a reception module 117 to generate ultrasound data by performing analog-to-digital conversion on reception signals received from the probe 20, and summing the reception signals converted to digital signals, in consideration of the positions and focal points of the plurality of transducers 115.

When the probe 20 is implemented as the 2D probe, the processor 120 may calculate a time delay value for digital beamforming for each of the plurality of subarrays included in the 2D transducer array. Also, the processor 120 may calculate a time delay value for analog beamforming for each of transducers included in any one of the plurality of subarrays. The processor 120 may control the analog beamformer and the digital beamformer to generate a transmission signal to be applied to each of the plurality of transducers 115, according to the time delay values for analog beamforming and the time delay values for digital beamforming. Also, the processor 120 may control the analog beamformer to sum signals received from the plurality of transducers 115 for each subarray, according to the time delay values for analog beamforming. Also, the processor 120 may control the ultrasound transceiving module 110 to perform analog-to-digital conversion on a result of summing the signals for each subarray. Also, the processor 120 may control the digital beamformer to generate ultrasound data by summing the signals converted to digital signals, according to the time delay values for digital beamforming.

The image processor 130 generates or processes an ultrasound image by using the generated ultrasound data.

The display 140 may display the generated ultrasound image, and various pieces of information processed by the ultrasound imaging device 40 or the probe 20. According to an implementation, the probe 20 or the ultrasound imaging device 40 may include one or more displays 140. Also, the display 140 may include a touch panel or a touch screen. Also, the display 140 may include a flexible display.

The processor 120 may control all operations of the ultrasound imaging device 40, and may control operations of elements of the ultrasound imaging device 40. The processor 120 may perform or control various operations or functions of the ultrasound imaging device 40 by executing a program or instructions stored in a memory 150. Also, the processor 120 may control operations of the ultrasound imaging device 40 by receiving a control signal from the input interface 170 or an external device.

The ultrasound imaging device 40 may include a communication module 160 and may be connected to an external device (e.g., the probe 20, a server, a medical device, or a portable device (e.g., a smart phone, a tablet PC, a wearable device, etc.)) via the communication module 160.

The communication module 160 may include one or more elements configured to enable communication with the external device. The communication module 160 may include, for example, at least one of a short-range communication module, a wired communication module, or a wireless communication module.

The communication module 160 may receive a control signal or data from the external device. The processor 120 may control an operation of the ultrasound imaging device 40, according to the control signal received via the communication module 160. Also, the processor 120 may transmit a control signal to the external device via the communication module 160, thereby controlling the external device according to the control signal. The external device may operate according to a control signal received from the ultrasound imaging device 40, or may process data received from the ultrasound imaging device 40.

A program or an application related to the ultrasound imaging device 40 may be installed in the external device. The program or the application installed in the external device may control the ultrasound imaging device 40, or may operate according to a control signal or data received from the ultrasound imaging device 40.

The external device may receive or download the program or the application related to the ultrasound imaging device 40 from the ultrasound imaging device 40, the probe 20, or a server, and may install and execute the program or the application in the external device. The ultrasound imaging device 40, the probe 20, or the server which provides the program or the application may include a recording medium that stores an instruction, a command, an installation file, an execution file, related data, or the like of the program or the application. The external device having the program or the application installed therein may be sold.

The memory 150 may store various pieces of data or programs for operating and controlling the ultrasound imaging device 40, input/output ultrasound data, ultrasound images, and the like.

The input interface 170 may receive a user input for controlling the ultrasound imaging device 40. For example, the user input may include, but is not limited to, an input of manipulating a button, a keypad, a mouse, a trackball, a jog switch, a knop, or the like, an input of touching a touch pad or a touch screen, a voice input, a motion input, a biometric information input (e.g., iris recognition or fingerprint recognition), and the like.

FIG. 1B is a control block diagram of the ultrasound imaging system 100 of a case in which the probe 20 is the wireless probe or the hybrid probe.

According to various embodiments, the ultrasound imaging device 40 of FIG. 1B may be substituted with the ultrasound imaging device 40 described with reference to FIG. 1A.

According to various embodiments, the probe 20 of FIG. 1A may be substituted with the probe 20 to be described with reference to FIG. 1B.

The probe 20 may include a display 112, the transmission module 113, a battery 114, a transducer 115, a charging module 116, a reception module 117, an input interface 109, a processor 118, and a communication module 119. FIG. 1B illustrates that the probe 20 includes all of the transmission module 113 and the reception module 117, but, according to an implementation, the probe 20 may include only a portion of configurations of the transmission module 113 and the reception module 117, and a portion of the configurations of the transmission module 113 and the reception module 117 may be included in the ultrasound imaging device 40. Also, according to an embodiment, the probe 20 may further include the image processor 130.

The transducer 115 may include a plurality of transducers. The plurality of transducers may be aligned in a preset array to be implemented as a transducer array. The transducer array may correspond to a 1D array or a 2D array. The plurality of transducers may transmit an ultrasound signal to an object 10, according to a transmission signal applied from a transmission module 113. The plurality of transducers may form or generate an electric reception signal by receiving an ultrasound signal reflected from the object 10.

The charging module 116 may charge the battery 114. The charging module 116 may receive power from an external source. According to an embodiment, the charging module 116 may wirelessly receive power. Also, according to an embodiment, the charging module 116 may receive power by wire. The charging module 116 may deliver the received power to the battery 114.

In consideration of positions and focal points of the plurality of transducers, the processor 118 controls the transmission module 113 to generate or form transmission signals to be respectively applied to the plurality of transducers.

The processor 118 controls the reception module 117 to generate ultrasound data by performing analog-to-digital conversion on reception signals received from the transducer 115, and summing the reception signals converted to digital signals, in consideration of the positions and focal points of the plurality of transducers. According to an embodiment, when the probe 20 includes the image processor 130, an ultrasound image may be generated by using the generated ultrasound data.

When the probe 20 is implemented as the 2D probe, the processor 118 may calculate a time delay value for digital beamforming for each of the plurality of subarrays included in the 2D transducer array. Also, the processor 118 may calculate a time delay value for analog beamforming for each of transducers included in any one of the plurality of subarrays. The processor 118 may control an analog beamformer and a digital beamformer to generate a transmission signal to be applied to each of the plurality of transducers, according to the time delay values for analog beamforming and the time delay values for digital beamforming. Also, the processor 118 may control the analog beamformer to sum signals received from the plurality of transducers for each subarray, according to the time delay values for analog beamforming. Also, the processor 118 may control the ultrasound transceiving module 110 to perform analog-to-digital conversion on a result of summing the signals for each subarray. Also, the processor 118 may control the digital beamformer to generate ultrasound data by summing the signals converted to digital signals, according to the time delay values for digital beamforming.

The processor 118 may control all operations of the probe 20, and may control operations of elements of the probe 20. The processor 118 may perform or control various operations or functions of the probe 20 by executing a program or instructions stored in a memory 111. Also, the processor 118 may control operations of the probe 20 by receiving a control signal from the input interface 109 of the probe 20 or an external device (e.g.: the ultrasound imaging device 40). Also, the processor 118 may control operations of the probe 20 by receiving a control signal from the input interface 109 or the external device. The input interface 109 may receive a user input for controlling the probe 20. For example, the user input may include, but is not limited to, an input of manipulating a button, a keypad, a mouse, a trackball, a jog switch, a knop, or the like, an input of touching a touch pad or a touch screen, a voice input, a motion input, a biometric information input (e.g., iris recognition or fingerprint recognition), and the like.

The display 112 may display an ultrasound image generated by the probe 20, an ultrasound image generated by processing ultrasound data generated in the probe 20, an ultrasound image received from the ultrasound imaging device 40, or various pieces of information processed by the ultrasound imaging system 100. Also, the display 112 may further display state information of the probe 20. The state information of the probe 20 may include at least one of device information of the probe 20, battery state information of the probe 20, frequency band information of the probe 20, output information of the probe 20, information indicating normality or abnormality of the probe 20, setting information of the probe 20, or temperature information of the probe 20.

According to an implementation, the probe 20 may include one or more displays 112. Also, the display 112 may include a touch panel or a touch screen. Also, the display 112 may include a flexible display.

The communication module 119 may wirelessly transmit the generated ultrasound data or the ultrasound image to the ultrasound imaging device 40 a wireless network. Also, the communication module 119 may receive a control signal and data from the ultrasound imaging device 40.

The ultrasound imaging device 40 may receive the ultrasound data or the ultrasound image from the probe 20.

In an embodiment, when the probe 20 includes the image processor 130 capable of generating an ultrasound image by using ultrasound data, the probe 20 may transmit ultrasound data, or an ultrasound image generated by the image processor 130 to the ultrasound imaging device 40.

In an embodiment, when the probe 20 does not include the image processor 130 capable of generating an ultrasound image by using ultrasound data, the probe 20 may transmit ultrasound data to the ultrasound imaging device 40. Ultrasound data may include ultrasound raw data, and an ultrasound image may indicate ultrasound image data.

The ultrasound imaging device 40 may include the processor 120, the image processor 130, the display 140, the memory 150, the communication module 160, and the input interface 170.

The image processor 130 generates or processes an ultrasound image by using ultrasound data received from the probe 20.

The display 140 may display an ultrasound image received from the probe 20, an ultrasound image generated by processing ultrasound data received from the probe 20, or various pieces of information processed by the ultrasound imaging system 100. According to an implementation, the ultrasound imaging device 40 may include one or more displays 140. Also, the display 140 may include a touch panel or a touch screen. Also, the display 140 may include a flexible display.

The processor 120 may control all operations of the ultrasound imaging device 40, and may control operations of elements of the ultrasound imaging device 40. The processor 120 may perform or control various operations or functions of the ultrasound imaging device 40 by executing a program or an application stored in a memory 150. Also, the processor 120 may control operations of the ultrasound imaging device 40 by receiving a control signal from the input interface 170 or an external device.

The ultrasound imaging device 40 may include the communication module 160 and may be connected to an external device (e.g., the probe 20, a server, a medical device, or a portable device (e.g., a smart phone, a tablet PC, a wearable device, etc.)) via the communication module 160.

The communication module 160 may include one or more elements configured to enable communication with the external device. The communication module 160 may include, for example, at least one of a short-range communication module, a wired communication module, or a wireless communication module.

The communication module 160 of the ultrasound imaging device 40 and the communication module 119 of the probe 20 may communicate by using a network or may communicate by using a short-range wireless communication method. For example, the communication module 160 of the ultrasound imaging device 40 and the communication module 119 of the probe 20 may communicate by using at least one of wireless data communication methods including wireless local area network (LAN), Wi-Fi, Bluetooth, ZigBee, Wi-Fi direct (WFD), infrared data association (IrDA), Bluetooth low energy (BLE), near field communication (NFC), wireless broadband Internet (WiBro), world interoperability for microwave access (WiMAX), shared wireless access protocol (SWAP), wireless gigabit alliance (WiGig), radio frequency (RF) communication, or 60-GHz mmWave short-range communication.

To this end, the communication module 160 of the ultrasound imaging device 40 and the communication module 119 of the probe 20 may each include at least one of a wireless LAN communication module, a Wi-Fi communication module, a Bluetooth communication module, a ZigBee communication module, a WFD communication module, an IrDA communication module, a BLE communication module, an NFC communication module, a WiBro communication module, a WiMAX communication module, a SWAP communication module, a WiGig communication module, a RF communication module, or a 60-GHz mmWave short-range communication module.

In an embodiment, the probe 20 may transmit device information (e.g.: identification (ID) information) of the probe 20 to the ultrasound imaging device 40 by using a first communication method (e.g.: BLE), and may be wirelessly paired with the ultrasound imaging device 40. Also, the probe 20 may transmit ultrasound data and/or an ultrasound image to the paired ultrasound imaging device 40.

The device information of the probe 20 may include various pieces of information related to a serial number, a model name, or a battery state of the probe 20.

The ultrasound imaging device 40 may receive the device information (e.g.: ID information) of the probe 20 from the probe 20 by using the first communication method (e.g.: BLE), and may be wirelessly paired with the probe 20. Also, the ultrasound imaging device 40 may transmit an activation signal to the paired probe 20, and may receive ultrasound data and/or an ultrasound image from the probe 20. Here, the activation signal may include a signal for controlling an operation of the probe 20.

In an embodiment, the probe 20 may transmit the device information (e.g.: ID information) of the probe 20 to the ultrasound imaging device 40 by using the first communication method (e.g.: BLE), and may be wirelessly paired with the ultrasound imaging device 40. Also, the probe 20 may transmit ultrasound data and/or an ultrasound image to the ultrasound imaging device 40 paired by the first communication method, by using a second communication method (e.g.: 60-GHz mmWave, Wi-Fi).

The ultrasound imaging device 40 may receive the device information (e.g.: ID information) of the probe 20 from the probe 20 by using the first communication method (e.g.: BLE), and may be wirelessly paired with the probe 20. Also, the ultrasound imaging device 40 may transmit an activation signal to the paired probe 20, and may receive ultrasound data and/or an ultrasound image from the probe 20 by using the second communication method (e.g.: 60-GHz mmWave, Wi-Fi).

According to an embodiment, the first communication method used to pair the probe 20 with the ultrasound imaging device 40 may have a frequency band lower than a frequency band of the second communication method used for the probe 20 to transmit ultrasound data and/or an ultrasound image to the ultrasound imaging device 40.

The display 140 of the ultrasound imaging device 40 may display user interfaces (Uls) indicating the device information of the probe 20. For example, the display 140 may display ID information of the probe 20, a pairing method indicating a method of pairing with the probe 20, a data communication state between the probe 20 and the ultrasound imaging device 40, a method of performing data communication with the ultrasound imaging device 40, a UI indicating a battery state of the probe 20, or the like.

When the probe 20 includes the display 112, the display 112 of the probe 20 may display a UI indicating the device information of the probe 20. For example, the display 112 may display ID information of the probe 20, a pairing method indicating a method of pairing with the probe 20, a data communication state between the probe 20 and the ultrasound imaging device 40, a method of performing data communication with the ultrasound imaging device 40, a UI indicating a battery state of the probe 20, or the like.

The communication module 160 may receive a control signal or data from the external device. The processor 120 may control an operation of the ultrasound imaging device 40, according to the control signal received via the communication module 160.

Also, the processor 120 may transmit a control signal to the external device via the communication module 160, thereby controlling the external device according to the control signal. The external device may operate according to a control signal received from the ultrasound imaging device 40, or may process data received from the ultrasound imaging device 40.

The external device may receive or download the program or the application related to the ultrasound imaging device 40 from the ultrasound imaging device 40, the probe 20, or a server, and may install and execute the program or the application in the external device. The ultrasound imaging device 40, the probe 20, or the server which provides the program or the application may include a recording medium that stores an instruction, a command, an installation file, an execution file, related data, or the like of the program or the application. The external device having the program or the application installed therein may be sold.

The memory 150 may store various pieces of data or programs for operating and controlling the ultrasound imaging device 40, input/output ultrasound data, ultrasound images, and the like.

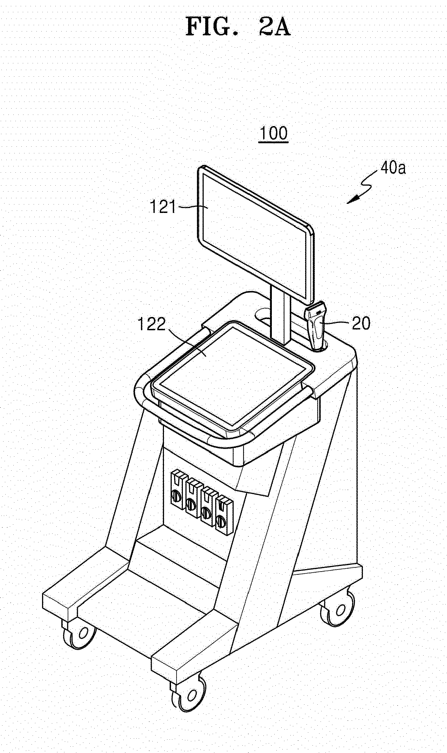

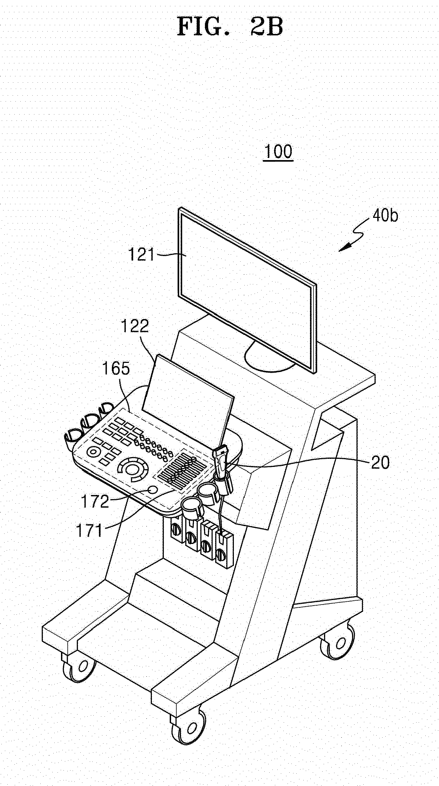

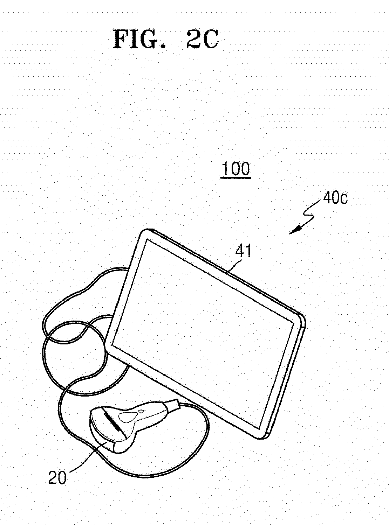

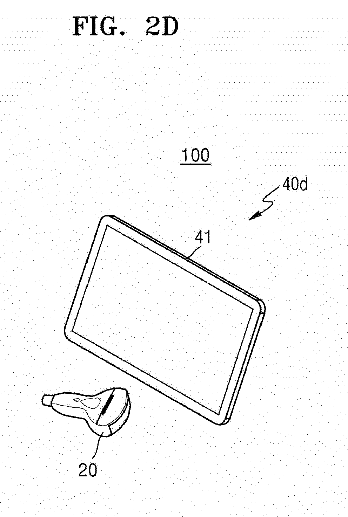

Examples of the ultrasound imaging system 100 according to an embodiment will now be described with reference to FIGS. 2A, 2B, 2C, and 2D.

FIGS. 2A, 2B, 2C, and 2D are diagrams illustrating ultrasound imaging devices according to an embodiment.

Referring to FIGS. 2A and 2B, each of ultrasound imaging devices 40a and 40b may include a main display 121 and a sub-display 122. Each of the main display 121 and the sub-display 122 may correspond to the display 140 of FIGS. 1A and 1B. At least one of the main display 121 or the sub-display 122 may be implemented as a touch screen. At least one of the main display 121 or the sub-display 122 may display an ultrasound image, or various pieces of information processed by the ultrasound imaging device 40a or 40b. Also, at least one of the main display 121 or the sub-display 122 may be implemented as a touch screen and may provide a graphical user interface (GUI), thereby receiving an input of data for controlling the ultrasound imaging device 40a or 40b from a user. For example, the main display 121 may display an ultrasound image, and the sub-display 122 may display a control panel in a GUI form so as to control a display of the ultrasound image. The sub-display 122 may receive an input of data for controlling a display of an image, via the control panel displayed in the GUI form. For example, a time gain compensation (TGC) button, a lateral gain compensation (LGC) button, a Freeze button, a trackball, a jog switch, a knop, or the like may be provided in the GUI form in the sub-display 122.

The ultrasound imaging device 40a or 40b may control a display of an ultrasound image displayed on the main display 121, by using input control data. Also, the ultrasound imaging device 40a or 40b may be connected to the probe 20 by wire or wirelessly, thereby transmitting or receiving an ultrasound signal to or from an object.

Referring to FIG. 2B, the ultrasound imaging device 40b may further include a control panel 165 as well as the main display 121 and the sub-display 122. The control panel 165 may include a button, a trackball, a jog switch, a knop, or the like, and may receive an input of data for controlling the ultrasound imaging device 40b from a user. For example, the control panel 165 may include a TGC button 171, a Freeze button 172, or the like. The TGC button 171 is a button for setting a TGC value for each depth of an ultrasound image. Also, when the ultrasound imaging device 40b detects an input to the Freeze button 172 while an ultrasound image is being scanned, the ultrasound imaging device 40b may maintain a state in which a frame image of a time point corresponding thereto is displayed, may capture the frame image of the time point, or may store the frame image of the time point.

The button, the trackball, the jog switch, the knop, or the like included in the control panel 165 may be provided in a GUI to the main display 121 or the sub-display 122. Also, the ultrasound imaging device 40a or 40b may be connected to the probe 20, thereby transmitting or receiving an ultrasound signal to or from an object.

Also, the ultrasound imaging device 40a or 40b may include an input/output interface of various types including as a speaker, a light-emitting diode (LED), a vibration device, or the like. For example, the ultrasound imaging device 40a or 40b may output, via the input/output interface, various pieces of information in the form of a graphic, sound, a vibration, or the like. Also, the ultrasound imaging device 40a or 40b may output, via the input/output interface, various notifications or data.

Referring to FIGS. 2C and 2D, the ultrasound imaging device 40a or 40b may be implemented to be portable. Examples of a portable ultrasound imaging device 40c or 40d may include, but are not limited to, a smart phone, a laptop computer, a PDA, a tablet PC, or the like which includes a probe and an application.

The portable ultrasound imaging device 40c may include a main body 41. Referring to FIG. 2C, the probe 20 may be connected by wire to one side of the main body 41. To this end, the main body 41 may include a connection terminal to which a cable connected to the probe 20 is detachable. The probe 20 may include a cable including a connection terminal that is connectable to the main body 41.

Referring to FIG. 2D, the probe 20 may be wirelessly connected to the ultrasound imaging device 40d. The main body 41 may include an input/output interface (e.g.: a touch screen). The input/output interface may display an ultrasound image, various pieces of information processed by an ultrasound imaging device, a GUI, or the like.

The ultrasound imaging device 40d and the probe 20 may establish communication or be paired with each other by using short-range wireless communication. For example, the ultrasound imaging device 40d and the probe 20 may perform communication by using Bluetooth, BLE, Wi-Fi, WFD, or the like.

The ultrasound imaging device 40c or 40d may control the probe 20 and output information related to the probe 20, by executing a program or an application related to the probe 20. The ultrasound imaging device 40c or 40d in communication with a preset server may perform an operation related to the probe 20. The probe 20 may be registered in the ultrasound imaging device 40c or 40d or may be registered in the preset server. The ultrasound imaging device 40c or 40d may communicate with the registered probe 20, and may perform an operation related to the probe 20.

Also, the ultrasound imaging device 40c or 40d may include an input/output interface of various types including as a speaker, a LED, a vibration device, or the like. For example, the ultrasound imaging device 40c or 40d may output, via the input/output interface, various pieces of information in the form of a graphic, sound, a vibration, or the like. Also, the ultrasound imaging device 40c or 40d may output, via the input/output interface, various notifications or data.

According to an embodiment, the ultrasound imaging device 40a, 40b, 40c, or 40d may process an ultrasound image or may obtain additional information from the ultrasound image, by using an artificial intelligence (Al) model. According to an embodiment, the ultrasound imaging device 40a, 40b, 40c, or 40d may generate an ultrasound image or perform processing such as correction, image-quality enhancement, encoding, decoding, or the like on an ultrasound image, by using the Al model. Also, according to an embodiment, the ultrasound imaging device 40a, 40b, 40c, or 40d may perform, from an ultrasound image, a definition of a reference line, obtainment of anatomical information, obtainment of lesion information, surface extraction, a boundary definition, length measurement, area measurement, volume measurement, annotation generation, or the like, by using the AI model.

The AI model may be arranged in the ultrasound imaging device 40a, 40b, 40c, or 40d or may be arranged in a server.

The AI model may be implemented by using various artificial neural networks or deep neural networks. Also, the AI model may be trained and generated by using various machine learning algorithms or deep learning algorithms. The AI model may be implemented by using a model including a convolutional neural network (CNN), a recurrent neural network (RNN), a generative adversarial network (GAN), a long short-term memory (LSTM), or the like.

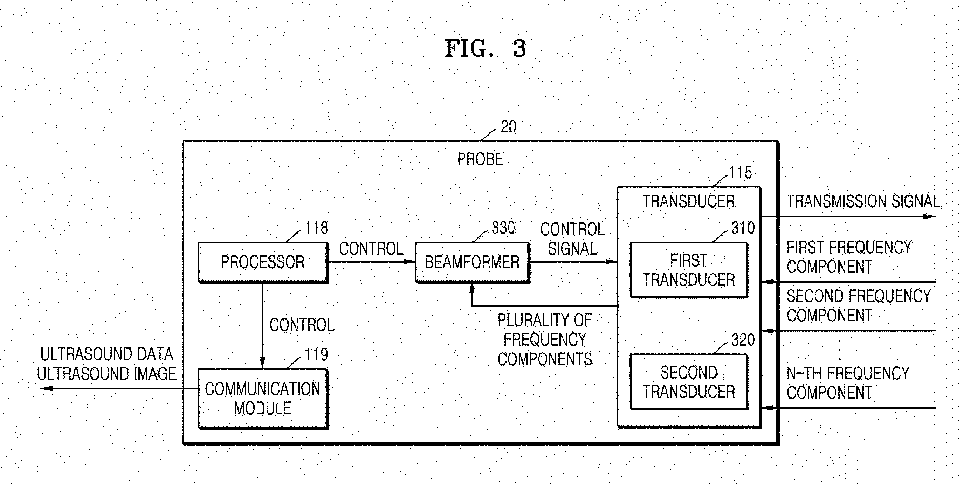

FIG. 3 is a block diagram of a probe 20 according to an embodiment. The probe 20 according to an embodiment may include a transducer 115, a beamformer 330, a processor 118, and a communication module 119. The transducer 115 of the probe 20 according to an embodiment may include a first transducer 310 and a second transducer 320. However, the disclosure is not limited thereto, and the probe 20 according to an embodiment may include a plurality of transducers 115.

Each of the first transducer 310 and the second transducer 320 may include a transducer array including a plurality of transducer units. The first transducer 310 and the second transducer 320 may be implemented to be physically separate. The first transducer 310 and the second transducer 320 may have parallel center axes. The center axis may be an axis that is at a center of a transducer and is vertical to a surface from which an ultrasound signal is emitted. Center axes of both a linear transducer and a convex transducer may each be at a center of a transducer and be vertical to a surface from which an ultrasound signal is emitted. When center axes of two transducers are parallel, scan regions of the two transducers may face in the same direction.

The first transducer 310 and the second transducer 320 which have parallel center axes may be arranged to be parallel to each other. For example, the first transducer 310 and the second transducer 320 may be arranged to be parallel to each other in a horizontal direction. For example, the first transducer 310 and the second transducer 320 may be arranged to be parallel to each other in a vertical direction.

The first transducer 310 and the second transducer 320 having parallel center axes may transmit and receive an ultrasound signal in the same direction. The transducer 115 including the first transducer 310 and the second transducer 320 may transmit a transmission signal. Also, the first transducer 310 and the second transducer 320 may receive an echo signal with respect to the transmission signal. When the transducer 115 including the first transducer 310 and the second transducer 320 receives an echo signal, the transducer 115 may receive a plurality of frequency components including a first frequency component, a second frequency component, ..., and a N-th frequency component (where, N is a natural number equal to or greater than 3). The plurality of frequency components may correspond to a plurality of frequency components included in the echo signal. The transducer 115 including the first transducer 310 and the second transducer 320 may relay the plurality of frequency components to the beamformer 330.

The first transducer 310 and the second transducer 320 may have different receivable bandwidths. The first transducer 310 may receive a signal of a low frequency band. For example, the first transducer 310 may receive a signal of about 1 MHz to about 10MHz. The second transducer 320 may receive a signal of a high frequency band. For example, the second transducer 320 may receive a signal of about 10MHz to about 15MHz.

The first transducer 310 and the second transducer 320 may have one structure or a seamlessly-combined semiconductor structure. The first transducer 310 and the second transducer 320 may be implemented in a capacitive micromachined ultrasonic transducer (cMUT) or piezoelectric micromachined ultrasonic transducer (pMUT) manner.

The beamformer 330 may control the first transducer 310 and the second transducer 320. The beamformer 330 may transmit a control signal to the transducer 115 including the first transducer 310 and the second transducer 320. The beamformer 330 may receive a plurality of frequency components from the transducer 115 including the first transducer 310 and the second transducer 320.

The probe 20 of an ultrasound imaging device may transmit a transmission signal to an object coated with a contrast agent, and may receive a reception signal reflected from the object. The reception signal reflected from the object coated with the contrast agent may include a plurality of frequency components. Due to a physical feature of the contrast agent, a plurality of harmonic components may occur when the reception signal is reflected. Compared to a tissue of the object, the contrast agent may have a wide-range of frequency signal components from sub-harmonic to N-th harmonic (where, N is a natural number equal to or greater than 3).

A bandwidth of a frequency each of the first transducer 310 and the second transducer 320 can receive may be physically limited. When an echo signal is received by using only one transducer among the first transducer 310 and the second transducer 320, only a frequency component of a bandwidth which is receivable by a used transducer among the first transducer 310 or the second transducer 320 is received from among a plurality of frequency components, and a frequency component of a bandwidth which is receivable by a not-used transducer may not be received. For example, a tissue image may further include a frequency component of a high frequency band. When only a frequency component of a low frequency band is received, a performance of the tissue image may deteriorate. For example, a contrast agent image may further include a frequency component of a low frequency band. When only a frequency component of a high frequency band is received, a performance of the contrast agent image may deteriorate.

The processor 118 may control the beamformer 330 and the communication module 119. The processor 118 may transmit a transmission signal by using at least one of the first transducer 310 or the second transducer 320. The processor 118 may receive a plurality of frequency components of an echo signal with respect to the transmission signal by using the first transducer 310 and the second transducer 320. The processor 118 may generate ultrasound data, based on the plurality of frequency components of the echo signal received by the first transducer 310 and the second transducer 320. When the processor 118 includes an image processor, the processor 118 may generate an ultrasound image based on the plurality of frequency components. The processor 118 may control the communication module 119 to transmit the ultrasound data to the ultrasound imaging device 40. When the processor 118 includes the image processor, the processor 118 may control the communication module 119 to transmit an ultrasound image to the ultrasound imaging device 40.

The communication module 119 may be controlled by the processor 118. The communication module 119 may transmit the ultrasound data to the ultrasound imaging device 40. The communication module 119 may transmit the ultrasound image to the ultrasound imaging device 40.

The probe 20 according to the disclosure may improve a quality of an ultrasound image by combining the transducers 310 and 320 of different species. After the probe 20 according to the disclosure transmits a transmission signal via one transducer, the probe 20 may receive a reception signal by simultaneously using a plurality of transducers. As the transducers 310 and 320 of different species are combined in the probe 20 according to the disclosure, the probe 20 may receive all of a plurality of frequency components that belong to frequency bands which are receivable by the transducers 310 and 320. The probe 20 according to the disclosure may receive all of a plurality of frequency components of an echo signal reflected from a contrast agent and a tissue. Accordingly, the probe 20 according to the disclosure may generate ultrasound data, based on frequency components of a wide band, thereby improving a quality of an ultrasound image displayed on an ultrasound diagnostic apparatus.

The processor 118 may control the first transducer 310 and the second transducer 320 by using the same control signal. The processor 118 may receive a plurality of frequency components by simultaneously using the first transducer 310 and the second transducer 320. The processor 118 may simultaneously activate the first transducer 310 and the second transducer 320 by using the same control signal. The processor 118 may control the beamformer 330 to transmit a single control signal to the transducer 115. The beamformer 330 may transmit the single control signal to the transducer 115, thereby simultaneously controlling the first transducer 310 and the second transducer 320.

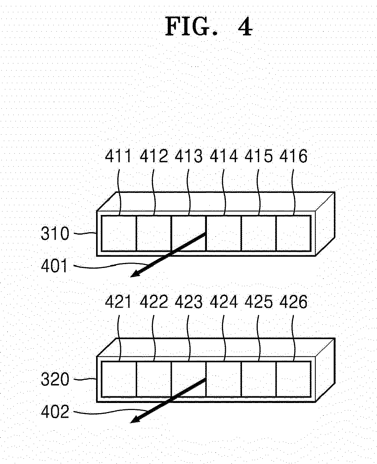

FIG. 4 is a diagram illustrating the first transducer 310 and the second transducer 320 of the probe 20, according to an embodiment.

The first transducer 310 and the second transducer 320 may have parallel center axes. A center axis may be an axis that is at a center of a transducer and is vertical to a surface from which an ultrasound signal is emitted. Center axes of both a linear transducer and a convex transducer may each be at a center of a transducer and be vertical to a surface from which an ultrasound signal is emitted. For example, the first transducer 310 may have a first center axis 401. For example, the second transducer 320 may have a second center axis 402. The first center axis 401 and the second center axis 402 may be parallel to each other. When center axes of two transducers are parallel, scan regions of the two transducers may face in the same direction.

The first transducer 310 and the second transducer 320 which have parallel center axes may be arranged to be parallel to each other. For example, the first transducer 310 and the second transducer 320 may be arranged to be parallel to each other in a horizontal direction. For example, the first transducer 310 and the second transducer 320 may be arranged to be parallel to each other in a vertical direction.

The first transducer 310 according to an embodiment may include a plurality of transducer units 411, 412, 413, 414, 415, and 416 which are arranged in an array structure. For example, the first transducer 310 may include a first transducer unit 411, a second transducer unit 412, a third transducer unit 413, a fourth transducer unit 414, a fifth transducer unit 415, and a sixth transducer unit 416. The plurality of transducer units 411, 412, 413, 414, 415, and 416 may be arranged in a single row.

The second transducer 320 according to an embodiment may include a plurality of transducer units 421, 422, 423, 424, 425, and 426 which are arranged in an array structure. For example, the second transducer 320 may include a first transducer unit 421, a second transducer unit 422, a third transducer unit 423, a fourth transducer unit 424, a fifth transducer unit 425, and a sixth transducer unit 426. The plurality of transducer units 421, 422, 423, 424, 425, and 426 may be arranged in a single row.

The first transducer 310 and the second transducer 320 may each be a piezoelectric entity configured to generate an ultrasound wave by converting an electric energy into a mechanical vibration. The first transducer 310 and the second transducer 320 may respectively receive frequency components that belong to different frequency bands. However, the disclosure is not limited thereto, and the first transducer 310 and the second transducer 320 may have one structure or a seamlessly-combined semiconductor structure. The first transducer 310 and the second transducer 320 may be implemented in a MUT or pMUT manner.

The probe 20 according to the disclosure may include a plurality of transducer units in an array structure. In the probe 20 according to the disclosure, the plurality of transducers 310 and 320 may be arranged with a light, slim, simple, and small form. Accordingly, different types of the transducers 310 and 320 may be easily combined with the probe 20 according to the disclosure.

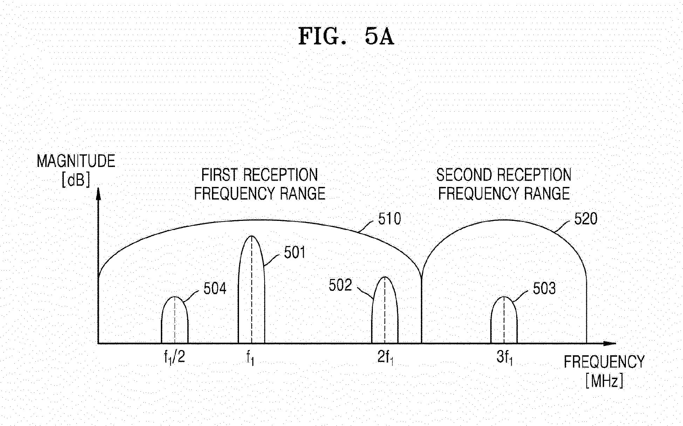

FIG. 5A is a diagram illustrating a plurality of frequency components received by the probe 20, a first reception frequency range of the probe 20, and a second reception frequency range of the probe 20, according to an embodiment.

The transducer 115 of the probe 20 according to an embodiment may transmit a transmission signal. For example, the first transducer 310 may transmit a transmission signal. A frequency of the transmission signal may be a first frequency f1. The first frequency f1 may be referred to as a fundamental frequency. According to an embodiment, the first transducer 310 may transmit an ultrasound signal of a frequency band or frequency range including the first frequency f1.

An object coated with a contrast agent may generate an echo signal including a plurality of frequency components while reflecting the transmission signal. The object coated with the contrast agent may generate the plurality of frequency components due to a non-linear characteristic of micro-bubbles formed in the contrast agent. For example, the object coated with the contrast agent may generate various types of frequency components including a fundamental frequency signal component, a N-th harmonics component (where, N is a natural number equal to or greater than 2), a 1/N-th sub-harmonics component (where, N is a natural number equal to or greater than 2), a M/2-th ultra-harmonics component (where, N is an odd number equal to or greater than 3), or the like.

The transducer 115 according to an embodiment may receive a plurality of frequency components 501, 502, 503, and 504. For example, the transducer 115 may receive the fundamental frequency signal 501, the second harmonic signal 502, the third harmonic signal 503, and the sub-harmonic signal 504. A frequency of the fundamental frequency signal 501 may be the first frequency f1. A frequency of the second harmonic signal 502 may be two times higher than the first frequency f1. A frequency of the third harmonic signal 503 may be three times higher than the first frequency f1. A frequency of the sub-harmonic signal 504 may be 0.5 times higher than the first frequency f1. However, the disclosure is not limited thereto, and the transducer 115 may receive a frequency component having a frequency lower than 0.5 times of the first frequency f1, or may receive a frequency component having a frequency higher than three times of the first frequency f1.

The first transducer 310 according to an embodiment may receive a signal of a first reception frequency range 510. The first reception frequency range 510 may be a low frequency range. For example, the first reception frequency range 510 may be a frequency range including the sub-harmonic signal 504, the fundamental frequency signal 501, and the second harmonic signal 502.

The second transducer 320 according to an embodiment may receive a signal of a second reception frequency range 520. The second reception frequency range 520 may be a high frequency range. For example, the second reception frequency range 520 may be a frequency range including the third harmonic signal 503.

The probe 20 according to an embodiment may receive the plurality of frequency components 501, 502, 503, and 504 by using the first transducer 310 and the second transducer 320. For example, the probe 20 may receive the sub-harmonic signal 504, the fundamental frequency signal 501, and the second harmonic signal 502 by using the first transducer 310. However, the disclosure is not limited thereto, and the probe 20 may receive the frequency component having the frequency lower than 0.5 times of the first frequency f1 by using the first transducer 310. For example, the probe 20 may receive the third harmonic signal 503 by using the second transducer 320. However, the disclosure is not limited thereto, and the probe 20 may receive the frequency component having the frequency higher than three times of the first frequency f1 by using the second transducer 320.

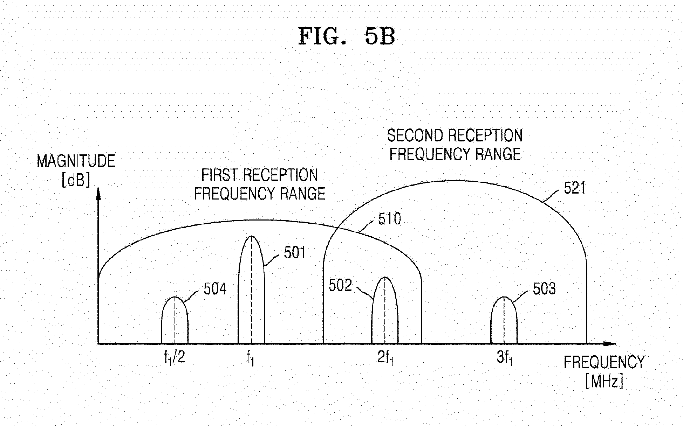

FIG. 5B is a diagram illustrating a plurality of frequency components received by the probe 20, a first reception frequency range of the probe 20, and a second reception frequency range of the probe 20, according to an embodiment.

The transducer 115 of the probe 20 according to an embodiment may transmit a transmission signal. For example, the transducer 115 may transmit a transmission signal having a first frequency f1 that is a fundamental frequency.

The transducer 115 according to an embodiment may receive a plurality of frequency components 501, 502, 503, and 504. For example, the transducer 115 may receive the fundamental frequency signal 501, the second harmonic signal 502, the third harmonic signal 503, and the sub-harmonic signal 504. However, the disclosure is not limited thereto, and the transducer 115 may receive a frequency component having a frequency lower than 0.5 times of the first frequency f1, or may receive a frequency component having a frequency higher than three times of the first frequency f1.

The first transducer 310 according to an embodiment may receive a signal of a first reception frequency range 510. The second transducer 320 according to an embodiment may receive a signal of a second reception frequency range 521. The second reception frequency range 521 may be a range including a middle frequency band and a high frequency band. For example, the second reception frequency range 521 may be a frequency range including the second harmonic signal 502 and the third harmonic signal 503.

According to an embodiment, the first reception frequency range 510 of the first transducer 310 and the second reception frequency range 521 of the second transducer 320 may partially overlap. For example, parts of the first reception frequency range 510 and the second reception frequency range 521 may overlap, and thus, both the first reception frequency range 510 and the second reception frequency range 521 may include the second harmonic signal 502.

The probe 20 according to an embodiment may receive the plurality of frequency components 501, 502, 503, and 504 by using the first transducer 310 and the second transducer 320. For example, the probe 20 may receive the sub-harmonic signal 504, the fundamental frequency signal 501, and the second harmonic signal 502 by using the first transducer 310, and may receive the second harmonic signal 502 and the third harmonic signal 503 by using the second transducer 320. However, the disclosure is not limited thereto, and the probe 20 may receive a frequency component having a frequency lower than 0.5 times of the first frequency f1 by using the first transducer 310, or may receive a frequency component having a frequency higher than three times of the first frequency f1 by using the second transducer 320.

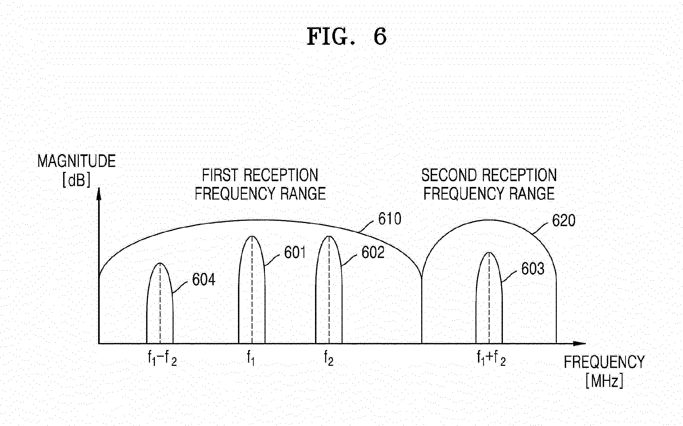

FIG. 6 is a diagram illustrating a plurality of frequency components received by the probe 20, a first reception frequency range of the probe 20, and a second reception frequency range of the probe 20, according to an embodiment.

The transducer 115 of the probe 20 according to an embodiment may transmit a plurality of transmission signals. The transducer 115 may simultaneously transmit a plurality of transmission signals with different frequencies via one of the first transducer 310 and the second transducer 320. For example, the first transducer 310 may transmit a first transmission signal. For example, the first transducer 310 may transmit a second transmission signal. A frequency of the first transmission signal may be a first frequency f1. A frequency of the second transmission signal may be a second frequency f2. The first transducer 310 may simultaneously transmit the first transmission signal and the second transmission signal.

An object may generate a plurality of frequency components while reflecting a transmission signal. When an ultrasound signal passes through a medium such as a tissue, the object may generate the plurality of frequency components due to a nonlinear propagation phenomenon and an inter-modulation distortion effect between multiple frequencies in a human tissue. For example, the object may generate various types of frequency components such as a first fundamental frequency component, a second fundamental frequency component, a sum frequency component, a difference frequency component, or the like. However, the disclosure is not limited thereto, and the object may generate various types of frequency components such as 2f2 - f1, 2f2 + f1, 2f2 - 2f1, 2f2 - 2f1, 3f1, 3f2, or the like.

The inter-modulation distortion effect may be a phenomenon in which, as an acoustic signal having two different frequencies moves within a medium, a distortion occurs in a waveform of the acoustic signal due to a non-linear characteristic of the medium, such that a sum frequency component and a difference frequency component are generated. When the acoustic signal having two different frequencies is mathematically modeled with a cosine function, a reflected acoustic signal may have a frequency component corresponding to a sum of frequencies of two cosine signal functions and a frequency component corresponding to a difference between the frequencies of the two cosine signal functions. The inter-modulation distortion effect may be similar to demodulation in a communication. The inter-modulation distortion effect may be referred to as self-demodulation as the acoustic signal is self-demodulated.

The transducer 115 according to an embodiment may receive a plurality of frequency components 601, 602, 603, and 604. For example, the transducer 115 may receive a first fundamental frequency signal 601, a second fundamental frequency signal 602, a sum frequency signal 603, and a difference frequency signal 604. A frequency of the first fundamental frequency signal 601 may be a first frequency f1. A frequency of the second fundamental frequency signal 602 may be a second frequency f2. A frequency of the sum frequency signal 603 may be a value obtained by adding the first frequency f1 and the second frequency f2. A frequency of the difference frequency signal 604 may be a value of a difference between the first frequency f1 and the second frequency f2.

The first transducer 310 according to an embodiment may receive a signal of a first reception frequency range 610. The first reception frequency range 610 may be a low frequency range. For example, the first reception frequency range 610 may be a frequency range including the difference frequency signal 604, the first fundamental frequency signal 601, and the second fundamental frequency signal 602. However, the disclosure is not limited thereto, and the object may receive frequency components that belong to the first reception frequency range 610 from among various types of frequency components such as 2f2 - f1, 2f2 - 2f1, 2f2 - 2f1, or the like.

The second transducer 320 according to an embodiment may receive a signal of a second reception frequency range 620. The second reception frequency range 620 may be a high frequency range. For example, the second reception frequency range 620 may be a frequency range including the sum frequency signal 603. However, the disclosure is not limited thereto, and the object may receive frequency components that belong to the second reception frequency range 620 from among various types of frequency components such as 2f2 + f1, 3f1, 3f2, or the like.

The probe 20 according to an embodiment may receive the plurality of frequency components 601, 602, 603, and 604 by using the first transducer 310 and the second transducer 320. For example, the probe 20 may receive the difference frequency signal 604, the first fundamental frequency signal 601, and the second fundamental frequency signal 602 by using the first transducer 310, and may receive the sum frequency signal 603 by using the second transducer 320. However, the disclosure is not limited thereto, and the probe 20 may receive various types of frequency components such as 2f2 - f1, 2f2 + f1, 2f2 - 2f1, 2f2 - 2f1, 3f1, 3f2, etc. by using the first transducer 310 and the second transducer 320.

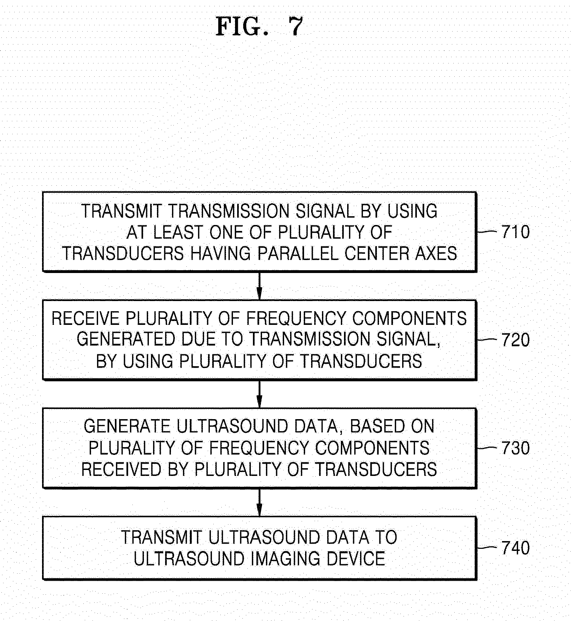

FIG. 7 is a flowchart illustrating a control method of the probe 20 according to an embodiment.

In operation 710, the probe 20 according to an embodiment may transmit a transmission signal by using at least one of a plurality of transducers having parallel center axes. The probe 20 according to an embodiment may combine transducers of different species. For example, the probe 20 may transmit a transmission signal by using one of the transducers of different species. However, the disclosure is not limited thereto, and the probe 20 may transmit the transmission signal by using at least two of the transducers of different species.

In operation 720, the probe 20 according to an embodiment may receive a plurality of frequency components generated due to the transmission signal, by using the plurality of transducers. The probe 20 according to an embodiment may receive, by using the plurality of transducers, the frequency components that are not receivable by one of the plurality of transducers. For example, the probe 20 may receive a plurality of frequency components by using both a transducer configured to receive a low frequency band and a transducer configured to receive a high frequency band.

In operation 730, the probe 20 according to an embodiment may generate ultrasound data, based on an echo signal of the plurality of frequency components received by the plurality of transducers. The probe 20 may generate the ultrasound data so as to generate an ultrasound image by using the received plurality of frequency components. When the probe 20 includes an image processor, the probe 20 may generate the ultrasound image by using the received plurality of frequency components.

In operation 740, the probe 20 according to an embodiment may transmit the ultrasound data to the ultrasound imaging device. The probe 20 may transmit the ultrasound data to the ultrasound imaging device, the ultrasound data being generated by using all of the plurality of frequency components. The ultrasound imaging device may display the ultrasound image to which all of the plurality of frequency components are applied. Accordingly, the probe 20 may improve a quality of the ultrasound image displayed by the ultrasound imaging device.

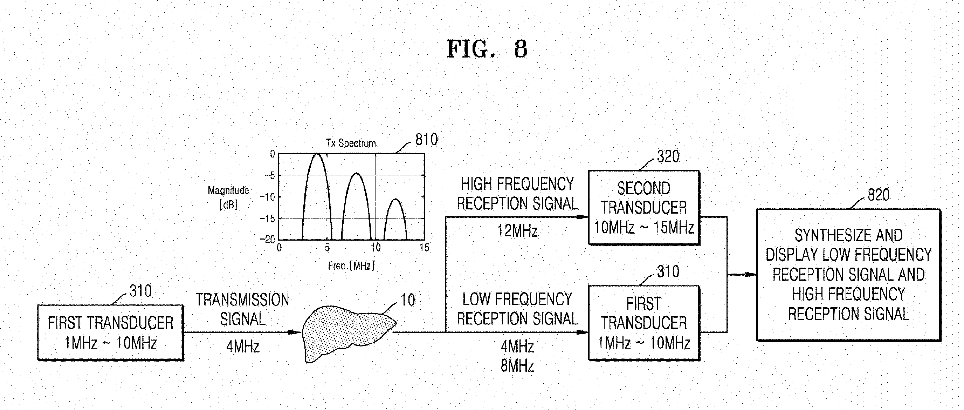

FIG. 8 illustrates that the probe 20 receives and synthesizes a low frequency reception signal and a high frequency reception signal due to a transmission signal of the first transducer 310 and displays a result of the synthesis, according to an embodiment.

The first transducer 310 according to an embodiment may transmit the transmission signal. The transmission signal transmitted by the first transducer 310 may have a first frequency. For example, the transmission signal transmitted by the first transducer 310 may have a frequency of 4MHz.

The transmission signal transmitted by the first transducer 310 may be reflected from an object 10. When an ultrasound signal is transmitted to an object, a plurality of reflection signal components may be generated due to a non-linear characteristic of the object. When the transmission signal is reflected from the object 10, a reception signal 810 including a plurality of frequency components may be generated. The reception signal 810 may include the plurality of frequency components that are generated based on the transmission signal. The reception signal 810 may include a fundamental frequency component and a multiple harmonic component. For example, the reception signal 810 may include a fundamental frequency signal component having a frequency of 4MHz, a two-multiple harmonic component having a frequency of 8MHz, and a three-multiple harmonic component having a frequency of 12MHz.

When the reception signal 810 is received by using only the first transducer 310, a signal of a high frequency component from among the plurality of frequency components may not be received. For example, when the reception signal 810 is received by using only the first transducer 310, the three-multiple harmonic component having the frequency of 12MHz from among the plurality of frequency components may not be received. Accordingly, when the reception signal 810 is received by using only the first transducer 310, the three-multiple harmonic component having the frequency of 12MHz may not be imaged.

The probe 20 may receive the reception signal 810 by using the first transducer 310 and the second transducer 320.

The first transducer 310 from among the plurality of transducers of the probe 20 according to an embodiment may receive a first frequency component included in a first reception frequency range from among the plurality of frequency components. The first transducer 310 may receive a low frequency reception signal. For example, the first transducer 310 may receive the fundamental frequency signal component having the frequency of 4MHz and the two-multiple harmonic component having the frequency of 8MHz.

The second transducer 320 from among the plurality of transducers of the probe 20 according to an embodiment may receive a second frequency component included in a second reception frequency range from among the plurality of frequency components. The second transducer 320 may receive a high frequency reception signal. For example, the second transducer 320 may receive the three-multiple harmonic component having the frequency of 12MHz.

The probe 20 according to an embodiment may synthesize the low frequency reception signal based on the first frequency component and the high frequency reception signal based on the second frequency component. The probe 20 may synthesize the low frequency reception signal received by the first transducer 310 and the high frequency reception signal received by the second transducer 320. The probe 20 may synthesize the low frequency reception signal and the high frequency reception signal by using a synthesis module 820.

The probe 20 according to an embodiment may generate ultrasound data by synthesizing the low frequency reception signal and the high frequency reception signal. The probe 20 may transmit the ultrasound data to the ultrasound imaging device. The probe 20 may transmit the ultrasound data to the ultrasound imaging device so as to allow the ultrasound imaging device to display an ultrasound image. However, the disclosure is not limited thereto, and the probe 20 may transmit the low frequency reception signal and the high frequency reception signal to the ultrasound imaging device, and then the ultrasound imaging device may synthesize the low frequency reception signal and the high frequency reception signal

The probe 20 according to the disclosure may combine a transducer configured to receive a low frequency band with a transducer configured to receive a high frequency band, thereby receiving all of a low frequency reception signal and a high frequency reception signal. The probe 20 according to the disclosure may receive all of frequencies that are not received when only one transducer is used. Accordingly, the probe 20 according to the disclosure may generate ultrasound data by using all of a low frequency reception signal and a high frequency reception signal, thereby improving a quality of an ultrasound image based on the ultrasound data.

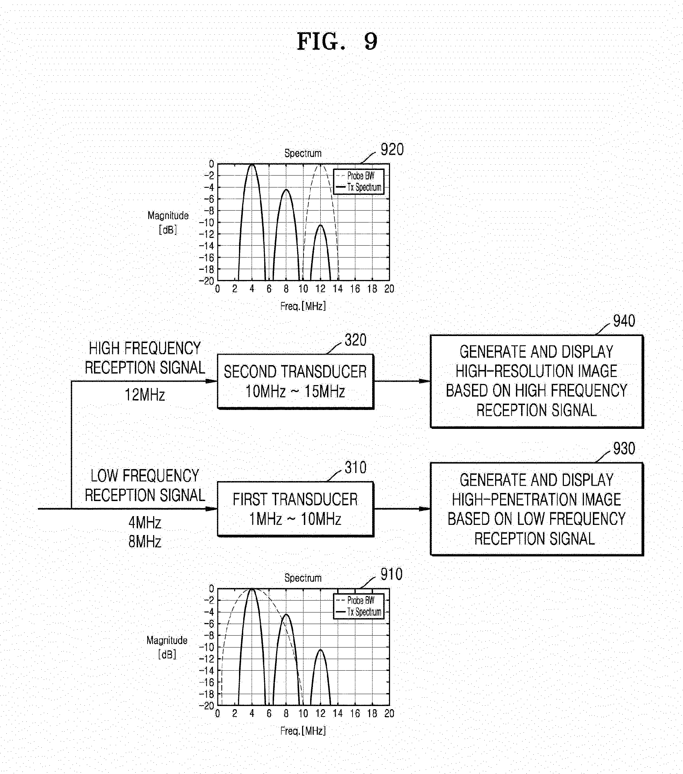

FIG. 9 illustrates that the probe 20 receives each of a low frequency reception signal and a high frequency reception signal, thereby generating and displaying different images, according to an embodiment.

The probe 20 according to an embodiment may receive a first reception signal 910 by using the first transducer 310. The first transducer 310 may receive a first frequency component included in a first reception frequency range from among a plurality of frequency components. For example, the first transducer 310 may receive a frequency component having a frequency of about 1 MHz to about 10MHz. The first transducer 310 may receive a low frequency reception signal. For example, the first transducer 310 may receive a fundamental frequency signal component having a frequency of 4MHz and a two-multiple harmonic component having a frequency of 8MHz.

The probe 20 according to an embodiment may receive a second reception signal 920 by using the second transducer 320. The second transducer 320 may receive a second frequency component included in a second reception frequency range from among the plurality of frequency components. For example, the second transducer 320 may receive a frequency component having a frequency of about 10MHz to about 15MHz. The second transducer 320 may receive a high frequency reception signal. For example, the second transducer 320 may receive a three-multiple harmonic component having a frequency of 12MHz.

The probe 20 according to an embodiment may generate a high-penetration image based on the low frequency reception signal based on the first frequency component. The high-penetration image may be an image that is generated by using a signal of a low frequency band and has a high sensitivity and a high penetration. The probe 20 may generate the high-penetration image based on the low frequency reception signal, by using a high-penetration image generation module 930.

The probe 20 according to an embodiment may generate a high-resolution image based on the high frequency reception signal based on the second frequency component. The high-resolution image may be an image that is generated by using a signal of a high frequency band and has a high resolution. The probe 20 may generate the high-resolution image based on the high frequency reception signal, by using a high-resolution image generation module 940.

The probe 20 according to an embodiment may transmit ultrasound data to the ultrasound imaging device. The probe 20 according to an embodiment may transmit the ultrasound data including the high-penetration image and the high-resolution image to the ultrasound imaging device so as to allow the ultrasound imaging device to display the high-penetration image and the high-resolution image.

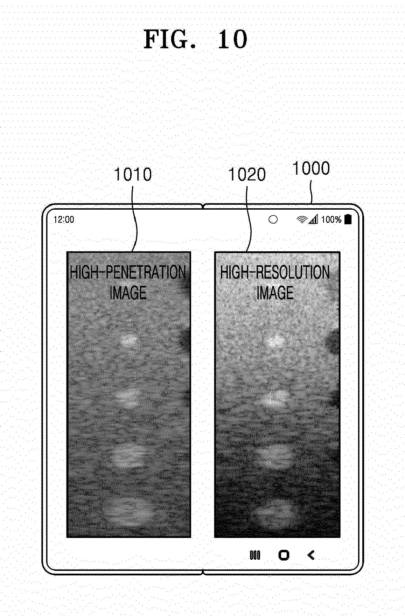

FIG. 10 illustrates an external device 1000, which received ultrasound data from the probe 20, displaying a high-penetration image 1010 and a high-resolution image 1020, according to an embodiment.

The probe 20 according to an embodiment may transmit the ultrasound data to the external device 1000. The external device 1000 may be an electronic device including a display. For example, the external device 1000 may be the ultrasound imaging device or the ultrasound diagnostic apparatus. For example, the external device 1000 may be a smartphone, a tablet, a PC, or the like. The external device 1000 may display an ultrasound image, based on the ultrasound data.

The external device 1000 may display the high-penetration image 1010 and the high-resolution image 1020, based on the ultrasound data. The external device 1000 may display the high-penetration image 1010, based on a low frequency component of the ultrasound data. The high-penetration image 1010 may include a contrast agent image. The external device 1000 may display the high-resolution image 1020, based on a high frequency component of the ultrasound data. The high-resolution image 1020 may include a tissue image.

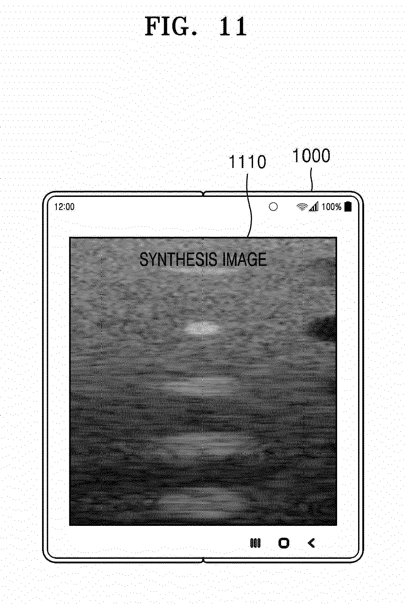

FIG. 11 illustrates the external device 1000, which received ultrasound data from the probe 20, displaying a synthesis image 1110, according to an embodiment.

The probe 20 may synthesize a low frequency component and a high frequency component included in a received plurality of frequency components. The probe 20 may generate ultrasound data including the low frequency component and the high frequency component. The ultrasound data generated by the probe 20 may have all of a high-penetration characteristic based on the low frequency component and a high-resolution characteristic based on the high frequency component.

The probe 20 according to an embodiment may transmit the ultrasound data to the external device 1000. The external device 1000 may be an electronic device including a display. For example, the external device 1000 may be the ultrasound imaging device or the ultrasound diagnostic apparatus. For example, the external device 1000 may be a smartphone, a tablet, a PC, or the like. The external device 1000 may display an ultrasound image, based on the ultrasound data.

The external device 1000 may display the synthesis image 1110, based on the ultrasound data. The synthesis image 1110 may have all of the high-penetration characteristic based on the low frequency component and the high-resolution characteristic based on the high frequency component. The external device 1000 may display the synthesis image 1110 in which a resolution and a speckle noise are enhanced, by synthesizing the low frequency component and the high frequency component.

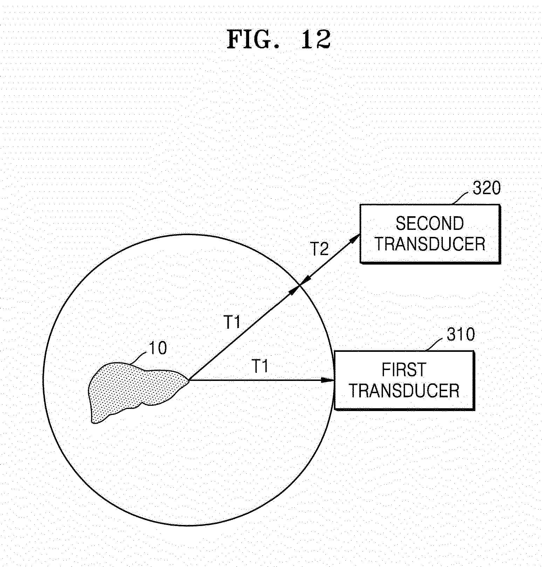

FIG. 12 illustrates that, when each of the first transducer 310 and the second transducer 320 of the probe 20 receive and synthesize a plurality of frequency components, a difference between a first time point of the first transducer 310 and a second time point of the second transducer 320 is compensated, according to an embodiment.

The probe 20 according to an embodiment may receive a reception signal reflected from the object 10, by using the first transducer 310 and the second transducer 320. The first transducer 310 may receive a low frequency reception signal included in the reception signal. The second transducer 320 may receive a high frequency reception signal included in the reception signal. The probe 20 may synthesize the received low frequency reception signal and the received high frequency reception signal.

The low frequency reception signal may be received by the first transducer 310 at the first time point after an elapse of a first time T1 after the reception signal is reflected from the object 10. The high frequency reception signal may be received by the second transducer 320 at the second time point after an elapse of a time that is a sum of the first time T1 and a second time T2 after the reception signal is reflected from the object 10.

When the probe 20 according to an embodiment synthesizes a low frequency reception signal and a high frequency reception signal, the probe 20 may compensate for a time difference between a first time point at which the first transducer 310 receives the low frequency reception signal and a second time point at which the second transducer 320 receives high frequency reception signal. The probe 20 may perform synthesis of the low frequency reception signal and the high frequency reception signal by applying that reception of the high frequency reception signal is delayed by the second time T2 that is the time difference between the first time point and the second time point.

Due to a physical distance between the first transducer 310 and the second transducer 320, a path in which the reception signal reflected from the object 10 travels to the first transducer 310 may be shorter than a path in which the reception signal travels to the second transducer 320. As the reception signal reflected from the object 10 is received late by the second transducer 320 than the first transducer 310, when the low frequency reception signal received by the first transducer 310 and the high frequency reception signal received by the second transducer 320 are synthesized, it is required to compensate for a difference between reception time points.

When the probe 20 according to an embodiment generates a synthesis image by using a low frequency reception signal and a high frequency reception signal, the probe 20 may apply a time compensation algorithm. For example, even when the reflection signal received from the object 10 reaches the first transducer 310 after the first time T1, the second time T2 may be additionally required for the same reception signal to reach the second transducer 320. The low frequency reception signal received by the first transducer 310 may be earlier than the high frequency reception signal received by the second transducer 320 by the second time T2. The probe 20 according to an embodiment may delay the low frequency reception signal by the second time T2, and then may perform synthesis of the low frequency reception signal and the high frequency reception signal.

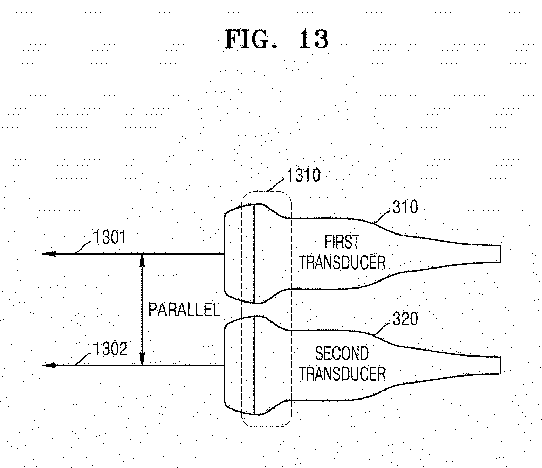

FIG. 13 illustrates a structure in which the first transducer 310 and the second transducer 320 of the probe 20 are combined to be parallel to each other, according to an embodiment.

The probe 20 according to an embodiment may include a combining module 1310. The combining module 1310 may combine the plurality of transducers 310 and 320 to be parallel to each other. FIG. 13 illustrates an example in which two transducers 310 and 320 are aligned to be horizontally combined to be parallel. However, the disclosure is not limited thereto, and the two transducers 310 and 320 may be aligned to be vertically combined to be parallel. That is, the two transducers 310 and 320 may be combined in a horizontal direction or a vertical direction by using the combining module 1310. For example, the combining module 1310 may be a holder to fix a first center axis 1301 of the first transducer 310 and a second center axis 1302 of the second transducer 320 to be parallel. The combining module 1310 may be coupled to the plurality of transducers 310 and 320 in such a manner that all of the plurality of transducers 310 and 320 may face in the same direction.

Each of the first transducer 310 and the second transducer 320 shown in FIG. 13 may be an independent probe. Accordingly, the structure of FIG. 13 in which two independent probes are combined may be implemented. In this case, each probe may include one transducer, one beamformer, one imaging device, and one communication module.

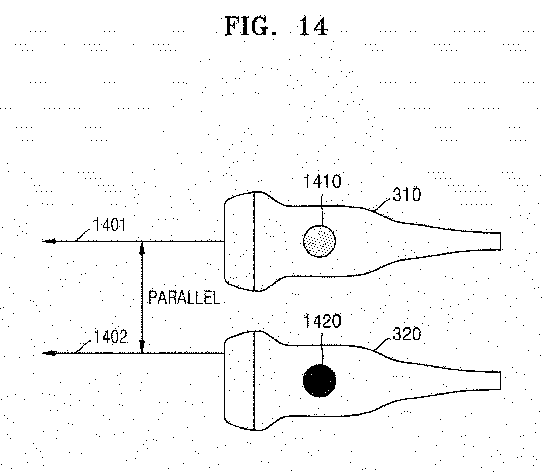

FIG. 14 illustrates a structure in which the first transducer 310 and the second transducer 320 of the probe are combined to be parallel, according to an embodiment.

The probe 20 according to an embodiment may include a plurality of combining modules 1410 and 1420 (hereinafter, also referred to as the first and second combining modules 1410 and 1420). For example, the probe 20 may include the first combining module 1410 provided at the first transducer 310 and the second combining module 1420 provided at the second transducer 320. For example, the first combining module 1410 and the second combining module 1420 may be combined to be detachable from the first transducer 310 and the second transducer 320.

The plurality of combining modules 1410 and 1420 according to an embodiment may be arranged with structures and at positions of the first transducer 310 and the second transducer 320 so as to correspond to each other. For example, the first combining module 1410 may be a female connector provided at a center part of the first transducer 310. For example, the second combining module 1420 may be a male connector provided at a center part of the second transducer 320 so as to correspond to the center part at which the first combining module 1410 is provided.

The plurality of combining modules 1410 and 1420 according to an embodiment may combine the plurality of transducers 310 and 320 to be parallel to each other. FIG. 14 illustrates an example in which two transducers 310 and 320 are aligned to be vertically combined to be parallel. However, the disclosure is not limited thereto, and the two transducers 310 and 320 may be aligned to be horizontally combined to be parallel. That is, the two transducers 310 and 320 may be combined in a vertical direction or a horizontal direction by using the first combining module 1410 and the second combining module 1420. For example, the first combining module 1410 and the second combining module 1420 may each be a bolt, a nut, or a magnet to fix a first center axis 1401 of the first transducer 310 and a second center axis 1402 of the second transducer 320 to be parallel. The plurality of combining modules 1410 and 1420 may be coupled to the plurality of transducers 310 and 320 in such a manner that all of the plurality of transducers 310 and 320 may face in the same direction.

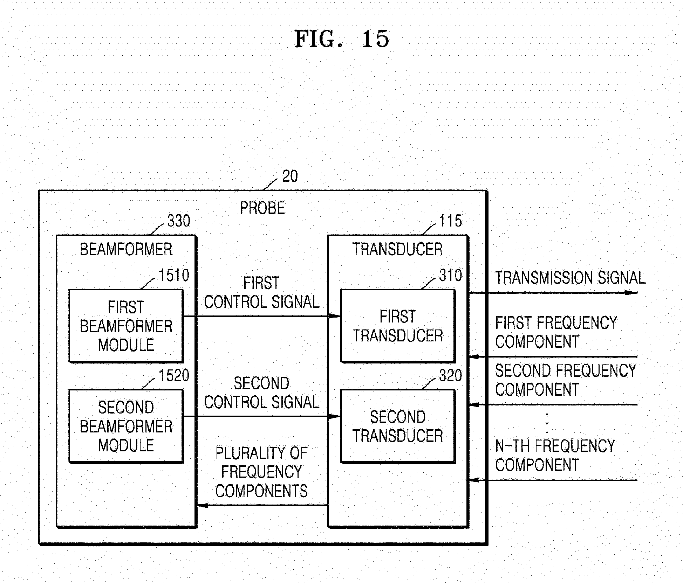

FIG. 15 is a block diagram illustrating that two beamformer modules 1510 and 1520 included in the beamformer 330 of the probe 20 respectively control two transducers 310 and 320, according to an embodiment.

The beamformer 330 according to an embodiment may include the beamformer module 1510 (also referred to as the first beamformer module 1510) connected to the first transducer 310 from among the plurality of transducers 310 and 320 and the beamformer module 1520 (also referred to as the second beamformer module 1520) connected to the second transducer 320 from among the plurality of transducers 310 and 320.

The first beamformer module 1510 may control the first transducer 310. The first beamformer module 1510 may transmit a first control signal to the first transducer 310.

The second beamformer module 1520 may control the second transducer 320. The second beamformer module 1520 may transmit a second control signal to the second transducer 320.

The transducer 115 including the first transducer 310 and the second transducer 320 may transmit a transmission signal. The transducer 115 including the first transducer 310 and the second transducer 320 may receive a plurality of frequency components including a first frequency component, a second frequency component, ..., and a N-th frequency component (where, N is a natural number equal to or greater than 3). The plurality of frequency components may be generated due to the transmission signal. The transducer 115 including the first transducer 310 and the second transducer 320 may relay the plurality of frequency components to the beamformer 330 including the first beamformer module 1510 and the second beamformer module 1520.

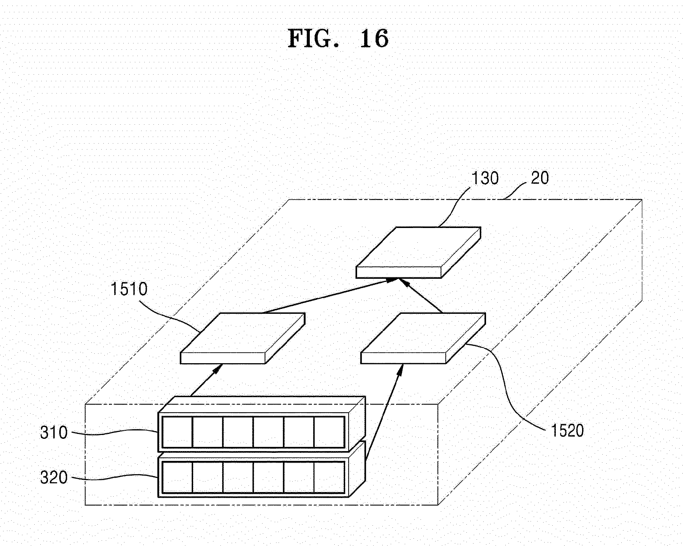

FIG. 16 is a block diagram illustrating that the two beamformer modules 1510 and 1520 included in the beamformer 330 of the probe 20 respectively control the two transducers 310 and 320, according to an embodiment.

The probe 20 according to an embodiment may include the first beamformer module 1510 and the second beamformer module 1520. The first beamformer module 1510 may connect the first transducer 310 and the image processor 130 with each other. The second beamformer module 1520 may connect the second transducer 320 and the image processor 130 with each other. The probe 20 according to an embodiment may control the first transducer 310 by using the first beamformer module 1510 and may control the second transducer 320 by using the second beamformer module 1520.

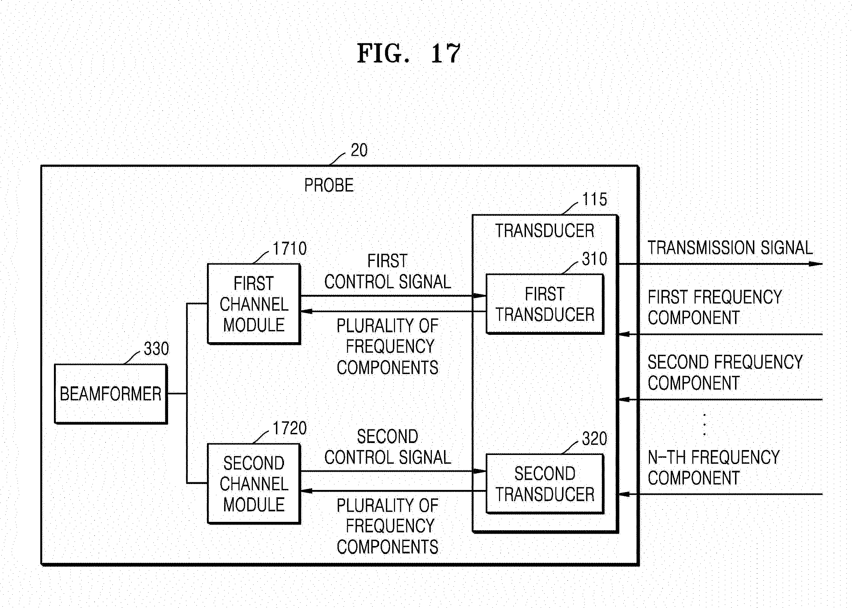

FIG. 17 is a block diagram illustrating that a first channel module 1710 and a second channel module 1720 which are connected to the beamformer 330 of the probe 20 respectively control the two transducers 310 and 320, according to an embodiment.

The first channel module 1710 may connect the beamformer 330 to the first transducer 310 from among the plurality of transducers 310 and 320. For example, the first channel module 1710 may include 1 to 64 channels from among 128 channels. The first channel module 1710 may connect the beamformer 330 to the first transducer 310 by using the 1 to 64 channels.

The second channel module 1720 may connect the beamformer 330 to the second transducer 320 from among the plurality of transducers 310 and 320. For example, the second channel module 1720 may include 65 to 128 channels from among the 128 channels. The second channel module 1720 may connect the beamformer 330 to the second transducer 320 by using the 65 to 128 channels.

The transducer 115 including the first transducer 310 and the second transducer 320 may transmit a transmission signal. The transducer 115 including the first transducer 310 and the second transducer 320 may receive a plurality of frequency components including a first frequency component, a second frequency component, ..., and a N-th frequency component (where, N is a natural number equal to or greater than 3). The plurality of frequency components may be generated due to the transmission signal. The transducer 115 including the first transducer 310 and the second transducer 320 may relay the plurality of frequency components to the beamformer 330 via the first channel module 1710 and the second channel module 1720.

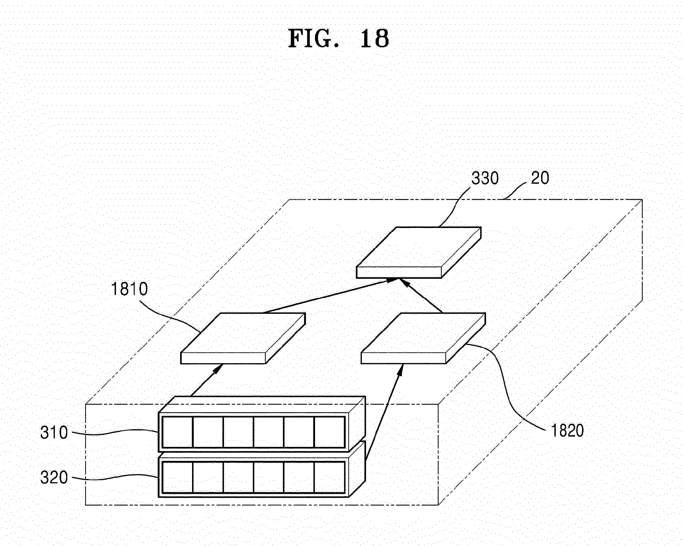

FIG. 18 is a block diagram illustrating that the first channel module 1710 and the second channel module 1720 which are connected to the beamformer 330 of the probe 20 respectively control the two transducers 310 and 320, according to an embodiment.

The probe 20 according to an embodiment may include a first multiplexer (MUX) 1810 and a second MUX 1820. The first MUX 1810 may include the first channel module 1710. The first MUX 1810 may connect the first transducer 310 and the beamformer 330 with each other. The second MUX 1820 may include the second channel module 1720. The second MUX 1820 may connect the second transducer 320 and the beamformer 330 with each other. The probe 20 according to an embodiment may control the first transducer 310 and the second transducer 320 by using one beamformer 330 connected to the first MUX 1810 and the second MUX 1820.

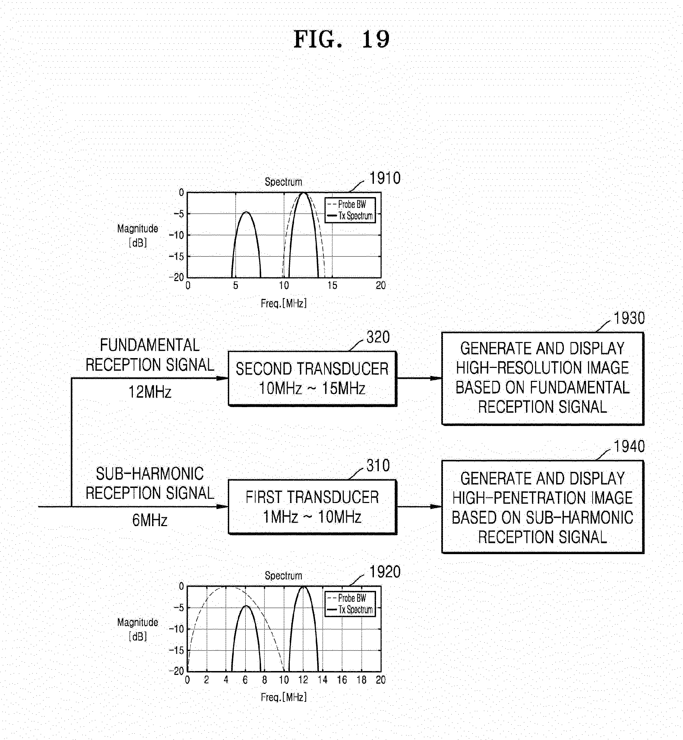

FIG. 19 illustrates that the probe 20 receives each of a fundamental reception signal and a sub-harmonic reception signal, and thus, generates and displays a high-resolution image and a high-penetration image, according to an embodiment.

The probe 20 according to an embodiment may receive a fundamental reception signal 1910 by using the second transducer 320. The second transducer 320 may receive the fundamental reception signal 1910, based on a second frequency component. For example, the second transducer 320 may receive a frequency component of about 10MHz to about 15MHz. For example, the second transducer 320 may receive the fundamental reception signal 1910 having a frequency of 12MHz.

The probe 20 according to an embodiment may receive a sub-harmonic reception signal 1920 by using the first transducer 310. The first transducer 310 may receive the sub-harmonic reception signal 1920, based on a first frequency component. The first transducer 310 may receive a frequency component having a frequency of about 1 MHz to about 10MHz. For example, the first transducer 310 may receive the sub-harmonic reception signal 1920 having a frequency of 6MHz.

The probe 20 according to an embodiment may generate a high-resolution image based on the fundamental reception signal 1910 based on the second frequency component. The high-resolution image may be an image that is generated by using a signal of a high frequency band and has a high resolution. The probe 20 may generate the high-resolution image based on the fundamental reception signal 1910, by using a high-resolution image generation module 1930.

The probe 20 according to an embodiment may generate a high-penetration image based on the sub-harmonic reception signal 1920 based on the first frequency component. The high-penetration image may be an image that is generated by using a signal of a low frequency band and has a high sensitivity and a high penetration. The probe 20 may generate the high-penetration image based on the sub-harmonic reception signal 1920, by using a high-penetration image generation module 1940.

The probe 20 according to an embodiment may transmit ultrasound data to the The probe 20 according to an embodiment may transmit the ultrasound data including the high-penetration image and the high-resolution image to the ultrasound imaging device so as to allow the ultrasound imaging device to display the high-penetration image and the high-resolution image.

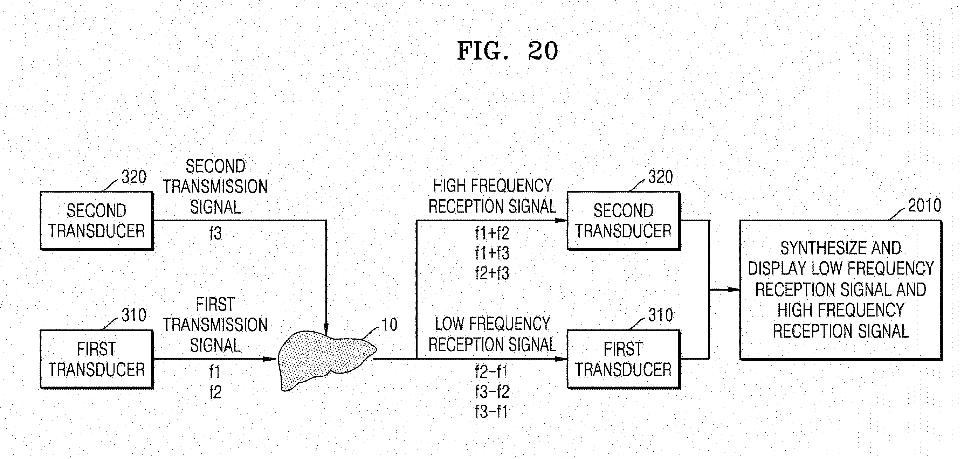

FIG. 20 illustrates that the probe 20 receives and synthesizes a low frequency reception signal and a high frequency reception signal due to a first transmission signal of the first transducer 310 and a second transmission signal of the second transducer 320 and displays a result of the synthesis, according to an embodiment.

The first transducer 310 according to an embodiment may transmit the first transmission signal. The first transmission signal transmitted by the first transducer 310 may have a first frequency f1 and a second frequency f2.

The second transducer 320 according to an embodiment may transmit a second transmission signal. The second transmission signal transmitted by the second transducer 320 may have a third frequency f3.

The first transmission signal and the second transmission signal transmitted by the first transducer 310 and the second transducer 320 according to an embodiment may be reflected from the object 10. When an ultrasound signal is transmitted to an object, a plurality of reflection signal components may be generated due to a non-linear characteristic of the object. When the first transmission signal and the second transmission signal are reflected from the object 10, a reception signal including a plurality of frequency components may be generated. The reception signal may include the plurality of frequency components generated due to the inter-modulation distortion effect. The reception signal may include a sum frequency component and a difference frequency component. For example, the reception signal may include frequency components of f2-f1, f3-f1, f3-f1, f1 +f2, f1+f3, and f2+f3.

The probe 20 may receive the reception signal by using the first transducer 310 and the second transducer 320.

The first transducer 310 from among the plurality of transducers of the probe 20 according to an embodiment may receive a first frequency component included in a first reception frequency range from among the plurality of frequency components. The first transducer 310 may receive a low frequency reception signal. For example, the first transducer 310 may receive the low frequency reception signal having frequencies of f2-f1, f3-f1, and f3-f2.

The second transducer 320 from among the plurality of transducers of the probe 20 according to an embodiment may receive a second frequency component included in a second reception frequency range from among the plurality of frequency components. The second transducer 320 may receive a high frequency reception signal. For example, the second transducer 320 may receive an inter-modulation signal component having frequencies of f1+f2, f1+f3, and f2+f3.

The probe 20 according to an embodiment may synthesize the low frequency reception signal based on the first frequency component and the high frequency reception signal based on the second frequency component. The probe 20 may synthesize the low frequency reception signal received by the first transducer 310 and the high frequency reception signal received by the second transducer 320. The probe 20 may synthesize the low frequency reception signal and the high frequency reception signal by using a synthesis module 2010.

The probe 20 according to an embodiment may generate ultrasound data by synthesizing the low frequency reception signal and the high frequency reception signal. The probe 20 may transmit the ultrasound data to the ultrasound imaging device. The probe 20 may transmit the ultrasound data to the ultrasound imaging device so as to allow the ultrasound imaging device to display an ultrasound image.

The probe 20 according to the disclosure may combine a transducer configured to receive a low frequency band and a transducer configured to receive a high frequency band, thereby receiving all of a low frequency reception signal and a high frequency reception signal. The probe 20 according to the disclosure may receive all of frequencies that are not received when only one transducer is used. Accordingly, the probe 20 according to the disclosure may generate ultrasound data by using all of a low frequency reception signal and a high frequency reception signal, thereby improving a quality of an ultrasound image based on the ultrasound data.

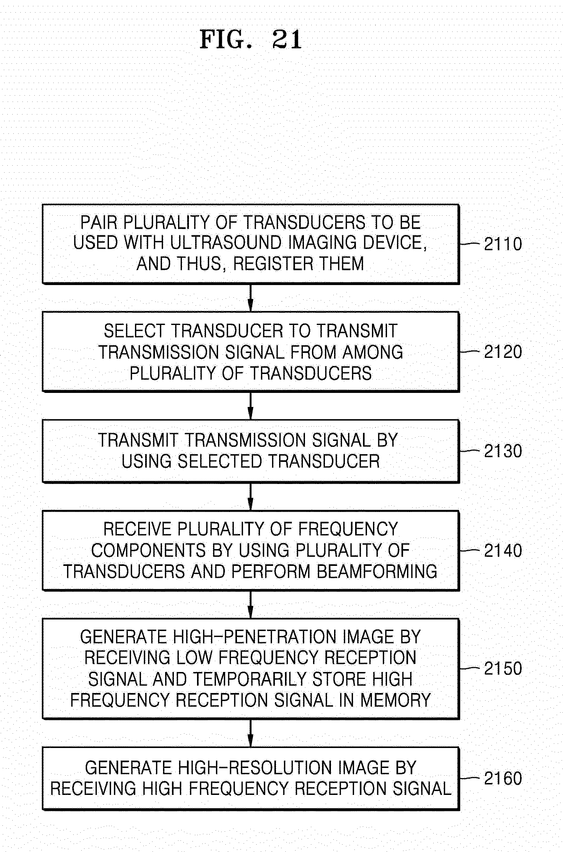

FIG. 21 is a flowchart illustrating that the probe 20 generates a high-penetration image and a high-resolution image, according to an embodiment.

In operation 2110, the probe 20 according to an embodiment may pair a plurality of transducers to be used with an ultrasound imaging device, and thus, register them.

In operation 2120, the probe 20 according to an embodiment may select a transducer to transmit a transmission signal from among the plurality of transducers.

In operation 2130, the probe 20 according to an embodiment may transmit a transmission signal by using the selected transducer.

In operation 2140, the probe 20 according to an embodiment may receive a plurality of frequency components by using the plurality of transducers and may perform beamforming.

In operation 2150, the probe 20 according to an embodiment may generate a high-penetration image by receiving a low frequency reception signal and may temporarily store a high frequency reception signal in a memory.

In operation 2160, the probe 20 according to an embodiment may generate a high-resolution image by receiving the high frequency reception signal.

The disclosure provides a probe configured to receive all of a plurality of frequency components included in a reception signal reflected from an object, and to generate ultrasound data capable of displaying an ultrasound image of an improved quality, by using the received plurality of frequency components, and a control method of the probe.

According to an embodiment, the probe 20 may include the plurality of transducers 310 and 320 having parallel center axes and configured to receive an ultrasound signal of different frequency ranges, the beamformer 330 configured to control the plurality of transducers 310 and 320, the communication module 119, and the processor 118. According to an embodiment, the processor 118 may be configured to transmit a transmission signal by using at least one of the plurality of transducers 310 and 320. According to an embodiment, the processor 118 may be configured to receive, by using the plurality of transducers 310 and 320, a plurality of frequency components that are generated due to the transmission signal and are included in the different frequency ranges. According to an embodiment, the processor 118 may be configured to generate ultrasound data, based on the plurality of frequency components received by the plurality of transducers 310 and 320. According to an embodiment, the processor 118 may be configured to control the communication module 119 to transmit the ultrasound data to the ultrasound imaging device.

According to an embodiment, the processor 118 may be configured to control the plurality of transducers 310 and 320 by using a same control signal.

According to an embodiment, each of the plurality of transducers 310 and 320 may include a plurality of transducer units arranged in an array structure.

According to an embodiment, the first transducer 310 from among the plurality of transducers 310 and 320 may be configured to receive a first frequency component included in a first reception frequency range from among the plurality of frequency components. According to an embodiment, the second transducer 320 from among the plurality of transducers 310 and 320 is configured to receive a second frequency component included in a second reception frequency range from among the plurality of frequency components.

According to an embodiment, the processor 118 may be configured to synthesize a low frequency reception signal based on the first frequency component and a high frequency reception signal based on the second frequency component. According to an embodiment, the processor 118 may be configured to control the ultrasound imaging device to display an ultrasound image by transmitting the ultrasound data to the ultrasound imaging device, the ultrasound data being generated by synthesizing the low frequency reception signal and the high frequency reception signal.

According to an embodiment, the processor 118 may be configured to generate a high-penetration image, based on a low frequency reception signal based on the first frequency component. According to an embodiment, the processor 118 may be configured to generate a high-resolution image, based on a high frequency reception signal based on the second frequency component. According to an embodiment, the processor 118 may be configured to control the ultrasound imaging device to display the high-penetration image and the high-resolution image, by transmitting the ultrasound data including the high-penetration image and the high-resolution image to the ultrasound imaging device.

According to an embodiment, the processor 118 may be configured to, when synthesizing the low frequency reception signal and the high frequency reception signal, compensate for a time difference between a first time point at which the first transducer 310 receives the low frequency reception signal and a second time point at which the second transducer 320 receives the high frequency reception signal.

According to an embodiment, the probe 20 may include the at least one combining module 1310 configured to combine the plurality of transducers to be parallel to each other.

According to an embodiment, the beamformer 330 may include the first beamformer module 1510 connected to the first transducer 320 from among the plurality of transducers 310 and 320. According to an embodiment, the beamformer 330 may include the second beamformer module 1520 connected to the second transducer 320 from among the plurality of transducers 310 and 320.

According to an embodiment, the probe 20 may include the first channel module 1710 configured to connect the beamformer 330 to the first transducer 310 from among the plurality of transducers 310 and 320. According to an embodiment, the probe 20 may include the second channel module 1720 configured to connect the beamformer 330 to the second transducer 320 from among the plurality of transducers 310 and 320.

According to an embodiment, the processor 118 may be configured to generate a high-penetration image, based on a sub-harmonic reception signal based on the first frequency component. According to an embodiment, the processor 118 may be configured to generate a high-resolution image, based on a fundamental reception signal based on the second frequency component. According to an embodiment, the processor 118 may be configured to control the ultrasound imaging device to display the high-penetration image and the high-resolution image, by transmitting the ultrasound data including the high-penetration image and the high-resolution image to the ultrasound imaging device.

According to an embodiment, the processor 118 may be configured to transmit a first transmission signal by using the first transducer 310. According to an embodiment, the processor 118 may be configured to transmit a second transmission signal by using the second transducer 320. According to an embodiment, the processor 118 may be configured to receive, by using the first transducer 310, the first frequency component as a low frequency reception signal from among the plurality of frequency components generated based on the first transmission signal and the second transmission signal. According to an embodiment, the processor 118 may be configured to receive, by using the second transducer 320, the second frequency component as a high frequency reception signal from among the plurality of frequency components generated based on the first transmission signal and the second transmission signal.

According to an embodiment, a control method of a probe may include transmitting, by the probe, a transmission signal by using at least one of a plurality of transducers having parallel center axes and configured to receive an ultrasound signal of different frequency ranges. According to an embodiment, the control method may include receiving, by the probe using the plurality of transducers, a plurality of frequency components that are generated due to the transmission signal and are included in the different frequency ranges. According to an embodiment, the control method may include generating, by the probe, ultrasound data, based on the plurality of frequency components received by the plurality of transducers. According to an embodiment, the control method may include transmitting, by the probe, the ultrasound data to an ultrasound imaging device.

According to an embodiment, the receiving of the plurality of frequency components may include receiving, by a first transducer from among the plurality of transducers, a first frequency component included in a first reception frequency range from among the plurality of frequency components. According to an embodiment, the receiving of the plurality of frequency components may include receiving, by a second transducer from among the plurality of transducers, a second frequency component included in a second reception frequency range from among the plurality of frequency components.

According to an embodiment, the generating of the ultrasound data may include synthesizing a low frequency reception signal based on the first frequency component and a high frequency reception signal based on the second frequency component. According to an embodiment, the transmitting to the ultrasound imaging device may include allowing the ultrasound imaging device to display an ultrasound image by transmitting the ultrasound data to the ultrasound imaging device, the ultrasound data being generated by synthesizing the low frequency reception signal and the high frequency reception signal.

According to an embodiment, the generating of the ultrasound data may include generating a high-penetration image, based on a low frequency reception signal based on the first frequency component. According to an embodiment, the generating of the ultrasound data may include generating a high-resolution image, based on a high frequency reception signal based on the second frequency component. According to an embodiment, the transmitting to the ultrasound imaging device may include allowing the ultrasound imaging device to display the high-penetration image and the high-resolution image, by transmitting the ultrasound data including the high-penetration image and the high-resolution image to the ultrasound imaging device.

According to an embodiment, the synthesizing of the low frequency reception signal and the high frequency reception signal may include compensating for a time difference between a first time point at which the first transducer receives the low frequency reception signal and a second time point at which the second transducer receives the high frequency reception signal.

According to an embodiment, the generating of the ultrasound data may include generating a high-penetration image, based on a sub-harmonic reception signal based on the first frequency component. According to an embodiment, the generating of the ultrasound data may include generating a high-resolution image, based on a fundamental reception signal based on the second frequency component. According to an embodiment, the transmitting to the ultrasound imaging device may include allowing the ultrasound imaging device to display the high-penetration image and the high-resolution image, by transmitting the ultrasound data including the high-penetration image and the high-resolution image to the ultrasound imaging device.

According to an embodiment, the transmitting of the transmission signal may include transmitting a first transmission signal by using the first transducer. According to an embodiment, the transmitting of the transmission signal may include transmitting a second transmission signal by using the second transducer. According to an embodiment, the receiving of the plurality of frequency components may include receiving, by using the first transducer, the first frequency component as a low frequency reception signal from among the plurality of frequency components generated based on the first transmission signal and the second transmission signal. According to an embodiment, the receiving of the plurality of frequency components may include receiving, by using the second transducer, the second frequency component as a high frequency reception signal from among the plurality of frequency components generated based on the first transmission signal and the second transmission signal.

According to an embodiment, the generating of the ultrasound data may include generating a high-penetration image by receiving a low frequency reception signal based on the first frequency component, and temporarily storing a high frequency reception signal based on the second frequency component in a memory. According to an embodiment, the generating of the ultrasound data may include generating a high-resolution image by receiving the high frequency reception signal.

According to an embodiment, the probe and the control method of the probe may generate ultrasound data by receiving all of a plurality of frequency components by using a plurality of transducers, thereby displaying an ultrasound image of an improved quality.

A device, a method, or a computer program according to an embodiment performs an operation related to an Al. The operation related to the AI is performed via a processor and a memory. The processor may perform the operation related to the AI by using one or more processors. In this case, the one or more processors may each be a general-purpose processor such as a central processing unit (CPU), an application processor (AP), a digital signal processor (DSP), or the like, a graphics-dedicated processor such as a graphics processing unit (GPU), a vision processing unit (VPU) or the like, or an Al-dedicated processor such as a neural processing unit (NPU). The one or more processors process input data, according to a program, an instruction, or an AI model stored in the memory.

The Al-related program, the instruction, or the AI model may be generated via machine learning. The meaning of being made via training indicates that a basic AI model is trained by using multiple training data based on a learning algorithm so as to execute desired characteristics (or purpose), thus making the predefined operation rule or AI model. Such training may be performed by a device on which AI according to an embodiment is implemented or by a separate server and/or a system. Examples of the learning algorithm may include, but are not limited to, supervised learning, unsupervised learning, semi-supervised learning, or reinforcement learning.

The AI model may include a plurality of neural network layers. Each of the plurality of neural network layers has a plurality of weight values, and performs a neural network operation through an operation between an operation result of a previous layer and the plurality of weight values. The plurality of weight values of the plurality of neural network layers may be optimized due to a training result of the AI model. Examples of the AI neural network may include, but are not limited to, a deep neural network (DNN), a convolutional neural network (CNN), a recurrent neural network (RNN), a restricted Boltzmann machine (RBM), a deep belief network (DBN), a bidirectional recurrent deep neural network (BRDNN), and a Deep Q-Networks (DQN).

A machine-readable storage medium may be provided in the form of a non-transitory storage medium. Here, the term "non-transitory storage medium" may mean that the storage medium is a tangible device and does not include signals (e.g., electromagnetic waves), and may mean that data may be permanently or temporarily stored in the storage medium. For example, the "non-transitory storage medium" may include a buffer in which data is temporality stored.

Furthermore, according to an embodiment, the method according to the embodiments may be provided in a computer program product. The computer program product may be traded between a seller and a purchaser as a commodity. The computer program product may be distributed in the form of the machine-readable storage medium (e.g., CD-ROM), or may be distributed online (e.g., downloaded or uploaded) through an application store or directly between two user devices (e.g., smart phones). For online distribution, at least a part of the computer program product (e.g., a downloadable app.) may be temporarily generated or be at least temporarily stored in a machine-readable storage medium such as a manufacturer's server, an application store's server, or a memory of a relay server.

## Claims

1. A probe comprising:
a plurality of transducers having parallel center axes and configured to receive an ultrasound signal of different frequency ranges;
a beamformer configured to control the plurality of transducers;
a communication module; and
a processor,
wherein the processor is configured to
transmit a transmission signal by using at least one of the plurality of transducers,
receive, by using the plurality of transducers, a plurality of frequency components that are generated due to the transmission signal and are comprised in the different frequency ranges,
generate ultrasound data, based on the plurality of frequency components received by the plurality of transducers, and
control the communication module to transmit the ultrasound data to an ultrasound imaging device.

2. The probe of claim 1, wherein the processor is further configured to control the plurality of transducers by using a same control signal.

3. The probe of claim 1, wherein each of the plurality of transducers comprises a plurality of transducer units arranged in an array structure.

4. The probe of claim 1, wherein
a first transducer from among the plurality of transducers is configured to receive a first frequency component comprised in a first reception frequency range from among the plurality of frequency components, and
a second transducer from among the plurality of transducers is configured to receive a second frequency component comprised in a second reception frequency range from among the plurality of frequency components.

5. The probe of claim 4, wherein the processor is further configured to
synthesize a low frequency reception signal based on the first frequency component and a high frequency reception signal based on the second frequency component, and
control the ultrasound imaging device to display an ultrasound image by transmitting the ultrasound data to the ultrasound imaging device, the ultrasound data being generated by synthesizing the low frequency reception signal and the high frequency reception signal.

6. The probe of claim 4, wherein the processor is further configured to
generate a high-penetration image, based on a low frequency reception signal based on the first frequency component,
generate a high-resolution image, based on a high frequency reception signal based on the second frequency component, and
control the ultrasound imaging device to display the high-penetration image and the high-resolution image, by transmitting the ultrasound data comprising the high-penetration image and the high-resolution image to the ultrasound imaging device.

7. The probe of claim 5, wherein the processor is further configured to,
when synthesizing the low frequency reception signal and the high frequency reception signal, compensate for a time difference between a first time point at which the first transducer receives the low frequency reception signal and a second time point at which the second transducer receives the high frequency reception signal.

8. The probe of claim 1, further comprising:
at least one combining module configured to combine the plurality of transducers to be parallel to each other.

9. The probe of claim 1, wherein the beamformer comprises:
a first beamformer module connected to a first transducer from among the plurality of transducers; and
a second beamformer module connected to a second transducer from among the plurality of transducers.

10. The probe of claim 1, further comprising:
a first channel module configured to connect the beamformer to the first transducer from among the plurality of transducers; and
a second channel module configured to connect the beamformer to the second transducer from among the plurality of transducers.

11. The probe of claim 4, wherein the processor is further configured to
generate a high-penetration image, based on a sub-harmonic reception signal based on the first frequency component,
generate a high-resolution image, based on a fundamental reception signal based on the second frequency component, and
control the ultrasound imaging device to display the high-penetration image and the high-resolution image, by transmitting the ultrasound data comprising the high-penetration image and the high-resolution image to the ultrasound imaging device.

12. The probe of claim 4, wherein the processor is further configured to
transmit a first transmission signal by using the first transducer,
transmit a second transmission signal by using the second transducer,
receive, by using the first transducer, the first frequency component as a low frequency reception signal from among the plurality of frequency components generated based on the first transmission signal and the second transmission signal, and
receive, by using the second transducer, the second frequency component as a high frequency reception signal from among the plurality of frequency components generated based on the first transmission signal and the second transmission signal.

13. A control method of a probe, the control method comprising:
transmitting, by the probe, a transmission signal by using at least one of a plurality of transducers having parallel center axes and configured to receive an ultrasound signal of different frequency ranges;
receiving, by the probe using the plurality of transducers, a plurality of frequency components that are generated due to the transmission signal and are comprised in the different frequency ranges;
generating, by the probe, ultrasound data, based on the plurality of frequency components received by the plurality of transducers; and
transmitting, by the probe, the ultrasound data to an ultrasound imaging device.

14. The control method of claim 13, wherein the receiving of the plurality of frequency components comprises:
receiving, by a first transducer from among the plurality of transducers, a first frequency component comprised in a first reception frequency range from among the plurality of frequency components; and
receiving, by a second transducer from among the plurality of transducers, a second frequency component comprised in a second reception frequency range from among the plurality of frequency components.

15. The control method of claim 14, wherein the generating of the ultrasound data comprises
generating a high-penetration image by receiving a low frequency reception signal based on the first frequency component, and temporarily storing a high frequency reception signal based on the second frequency component in a memory, and
generating a high-resolution image by receiving the high frequency reception signal.

## Amended claims

### Amended claims in accordance with Rule 137(2) EPC.

1. A probe (20) comprising:
a plurality of transducers (310, 320) having parallel center axes and configured to receive an ultrasound signal of different frequency ranges;
a beamformer (330) configured to control the plurality of transducers;
a communication module (119); and
a processor (118),
wherein the processor (118) is configured to
transmit a transmission signal by using at least one of the plurality of transducers (310, 320),
receive, by using the plurality of transducers (310, 320), a plurality of frequency components that are generated due to the transmission signal and are comprised in the different frequency ranges,
generate ultrasound data, based on the plurality of frequency components received by the plurality of transducers (310, 320), and
control the communication module (119) to transmit the ultrasound data to an ultrasound imaging device (40),
wherein the processor (118) is further configured to simultaneously activate a first transducer (310) and a second transducer (320) of the plurality of transducers (310, 320) by using a same control signal.

2. The probe (20) of claim 1, wherein each of the plurality of transducers (310, 320) comprises a plurality of transducer units arranged in an array structure.

3. The probe (20) of claim 1, wherein
a first transducer (310) from among the plurality of transducers (310, 320) is configured to receive a first frequency component comprised in a first reception frequency range from among the plurality of frequency components, and
a second transducer (320) from among the plurality of transducers (310, 320) is configured to receive a second frequency component comprised in a second reception frequency range from among the plurality of frequency components.

4. The probe (20) of claim 3, wherein the processor (118) is further configured to
generate a high-penetration image, based on a low frequency reception signal based on the first frequency component,
generate a high-resolution image, based on a high frequency reception signal based on the second frequency component, and
control the ultrasound imaging device (40) to display the high-penetration image and the high-resolution image, by transmitting the ultrasound data comprising the high-penetration image and the high-resolution image to the ultrasound imaging device (40).

5. The probe (20) of claim 3, wherein the processor (118) is further configured to
generate a high-penetration image, based on a sub-harmonic reception signal based on the first frequency component,
generate a high-resolution image, based on a fundamental reception signal based on the second frequency component, and
control the ultrasound imaging device (40) to display the high-penetration image and the high-resolution image, by transmitting the ultrasound data comprising the high-penetration image and the high-resolution image to the ultrasound imaging device (40).

6. The probe (20) of claim 3, wherein the processor (118) is further configured to
transmit a first transmission signal by using the first transducer (310),
transmit a second transmission signal by using the second transducer (320),
receive, by using the first transducer (310), the first frequency component as a low frequency reception signal from among the plurality of frequency components generated based on the first transmission signal and the second transmission signal, and
receive, by using the second transducer (320), the second frequency component as a high frequency reception signal from among the plurality of frequency components generated based on the first transmission signal and the second transmission signal.

7. The probe (20) of claim 3, wherein the processor (118) is further configured to
synthesize a low frequency reception signal based on the first frequency component and a high frequency reception signal based on the second frequency component, and
control the ultrasound imaging device (40) to display an ultrasound image by transmitting the ultrasound data to the ultrasound imaging device (40), the ultrasound data being generated by synthesizing the low frequency reception signal and the high frequency reception signal.

8. The probe (20) of claim 7, wherein the processor (118) is further configured to, when synthesizing the low frequency reception signal and the high frequency reception signal, compensate for a time difference between a first time point at which the first transducer (310) receives the low frequency reception signal and a second time point at which the second transducer (320) receives the high frequency reception signal.

9. The probe (20) of claim 1, further comprising:
at least one combining module (1310) configured to combine the plurality of transducers to be parallel to each other.

10. The probe (20) of claim 1, wherein the beamformer (330) comprises:
a first beamformer module (1510) connected to a first transducer (310) from among the plurality of transducers (310, 320); and
a second beamformer module (1520) connected to a second transducer (320) from among the plurality of transducers (310, 320).

11. The probe (20) of claim 1, further comprising:
a first channel module configured to connect the beamformer (330) to the first transducer (310) from among the plurality of transducers (310, 320); and
a second channel module configured to connect the beamformer (330) to the second transducer (320) from among the plurality of transducers (310, 320).

12. A control method of a probe (20), the control method comprising:
transmitting, by the probe (20), a transmission signal by using at least one of a plurality of transducers (310, 320) having parallel center axes and configured to receive an ultrasound signal of different frequency ranges;
receiving, by the probe (20) using the plurality of transducers (310, 320), a plurality of frequency components that are generated due to the transmission signal and are comprised in the different frequency ranges;
generating, by the probe (20), ultrasound data, based on the plurality of frequency components received by the plurality of transducers (310, 320); and
transmitting, by the probe (20), the ultrasound data to an ultrasound imaging device (40),
wherein the method further comprising:
simultaneously activate a first transducer (310) and a second transducer (320) of the plurality of transducers (310, 320) by using a same control signal.

13. The control method of claim 12, wherein the receiving of the plurality of frequency components comprises:
receiving, by a first transducer (310) from among the plurality of transducers (310, 320), a first frequency component comprised in a first reception frequency range from among the plurality of frequency components; and
receiving, by a second transducer (320) from among the plurality of transducers, a second frequency component comprised in a second reception frequency range from among the plurality of frequency components.

14. The control method of claim 13, wherein the generating of the ultrasound data comprises
generating a high-penetration image by receiving a low frequency reception signal based on the first frequency component, and temporarily storing a high frequency reception signal based on the second frequency component in a memory, and
generating a high-resolution image by receiving the high frequency reception signal.
